# EUROPEAN PATENT APPLICATION

(11) **EP 4 443 159 A1**
(43) Date of publication of application: **09.10.2024**
(21) Application number: 23166965.6
(22) Date of filing: 06.04.2023
(51) Int. Cl.: G01N 33/50, C07F 9/6558, C07F 9/6561, G01N 33/569, G01N 33/68

(54) **MEANS AND METHODS FOR DIAGNOSING A VIRAL INFECTION**

(71) Applicant: Bruker BioSpin GmbH & Co. KG, 76275 Ettlingen (DE); Murdoch University, Murdoch, WA 6150 (AU)
(72) Inventor: Wist, Julien, 6009 Perth WA (AU); Sala, Samuele, 6018 Woodlands WA (AU); Nitschke, Philipp, 6164 Yangebup WA (AU); Nicholson, Jeremy J., City Beach, WA 6015 (AU); Holmes, Elaine, City Beach, WA 6015 (AU); Trautwein, Christoph, 72138 Kirchentellinsfurt (DE); Cannet, Claire, 76275 Ettlingen (DE)
(74) Representative: Altmann Stößel Dick Patentanwälte PartG mbB

(57) **Abstract**

The invention relates to the field of viral diagnosis. In particular, it is directed to a method for diagnosing a viral infection or a disease associated therewith in a subject comprising the steps of: (a) determining in a sample of said subject the amount of at least one biomarker selected from the group of 3'(,5'-di)deoxy-3',4'-didehydro furanose derivatives of formula (I) with the exception of 3'-deoxy-3',4'-didehydro-cytidine (ddhC); and (b) comparing the amount(s) of the at least one biomarkers determined in (a) with a reference, thereby diagnosing a viral infection.

Further, the invention is directed to a method for diagnosing a viral infection or a disease associated therewith in a subject comprising the steps of: (A) determining in a sample of said subject the amount of 3'-deoxy-3',4'-didehydro-cytidine (ddhC); and (B) comparing the amount of the ddhC determined in (A) with a reference, thereby diagnosing a viral infection;
wherein the determination in (a) is done by NMR spectroscopy and the sample of the subject is a urine sample of said subject.

The invention is also related to a diagnostic means for use in diagnosing a viral infection or a disease associated therewith, said diagnostic means comprising at least one biomarker selected from the group of 3'(,5'-di)deoxy-3',4'-didehydro furanose derivatives of formula (I) with the exception of 3'-deoxy-3',4'-didehydro-cytidine (ddhC), but also to a diagnostic means comprising 3'-deoxy-3',4'-didehydro-cytidine (ddhC) for use in diagnosing a viral infection or a disease associated therewith, wherein the amount of the biomarker is determined done by NMR spectroscopy in a urine sample of a subject.

Furthermore, the invention is directed to a device for diagnosing a viral infection or a disease associated therewith in a sample of a subject and to a kit for identifying a subject suffering from a viral infection or a disease associated therewith.

## Description

The invention relates to the field of viral diagnosis. In particular, it is directed to a method for diagnosing a viral infection or a disease associated therewith in a subject comprising the steps of: (a) determining in a sample of said subject the amount of at least one biomarker selected from the group of 3'(,5'-di)deoxy-3',4'-didehydro furanose derivatives of formula (I) with the exception of 3'-deoxy-3',4'-didehydro-cytidine (ddhC); and (b) comparing the amount(s) of the at least one biomarkers determined in (a) with a reference, thereby diagnosing a viral infection.

Further, the invention is directed to a method for diagnosing a viral infection or a disease associated therewith in a subject comprising the steps of: (A) determining in a sample of said subject the amount of 3'-deoxy-3',4'-didehydro-cytidine (ddhC); and (B) comparing the amount of the ddhC determined in (A) with a reference, thereby diagnosing a viral infection;
wherein the determination in (a) is done by NMR spectroscopy and the sample of the subject is a urine sample of said subject.

The invention is also related to a diagnostic means for use in diagnosing a viral infection or a disease associated therewith, said diagnostic means comprising at least one biomarker selected from the group of 3'(,5'-di)deoxy-3',4'-didehydro furanose derivatives of formula (I) with the exception of 3'-deoxy-3',4'-didehydro-cytidine (ddhC), but also to a diagnostic means comprising 3'-deoxy-3',4'-didehydro-cytidine (ddhC) for use in diagnosing a viral infection or a disease associated therewith, wherein the amount of the biomarker is determined done by NMR spectroscopy in a urine sample of a subject.

Furthermore, the invention is directed to a device for diagnosing a viral infection or a disease associated therewith in a sample of a subject and to a kit for identifying a subject suffering from a viral infection or a disease associated therewith.

Due to the catastrophic impact on socioeconomic and healthcare systems, the disease COVID-19, and the etiological agent SARS-CoV-2, have been subjected to unprecedented clinical and biochemical scrutiny over the last three years. The disease has a strong respiratory phenotype, but multifaceted immunological consequences of the infection can affect all major organs with an associated diversity of physiological and clinical sub-phenotypes. The combination of these pathophysiological interactions can also lead to secondary onset of other forms of the disease, including phenoconversion into disease phenotypes reflecting cardiovascular, diabetic and neurological dysfunction. Thus, COVID-19 is an exceptionally complex systemic disease that requires integrated analytical platforms to reveal the underlying mechanistic processes. Encouragingly, by reciprocity, new insights about how SARS-CoV-2 infection drives long term consequences will impact research on separate diseases. Indeed, herein we report observations of a new family of molecules, nucleotide analogues, detected in the urine of SARS-CoV-2 infected patients that will increase our understanding of the natural defense mechanisms to deal with viruses.

Analytical platforms aiding the comprehensive metabolic phenotyping of biological fluids have the potential to deepen the understanding of the system-wide metabolic perturbations of disease, as well as facilitating the discovery of biomarkers that can assist with patient stratification and prognosis prediction. Amongst these techniques, liquid chromatography coupled to mass spectrometry (LC-MS) and Nuclear Magnetic Resonance (NMR) spectroscopy have emerged as two of the most powerful tools in the field of metabolic phenotyping and related disciplines: In particular, NMR spectroscopy of biological fluids has proven to be particularly advantageous due to its wide dynamic range and robustness, and as samples can be further reused for additional analyses¹.

Nucleotide analogues represent the largest class of clinically approved antiviral small molecules². Notable examples include the DNA polymerase inhibitor acyclovir, administered for the treatment of *Human alphaherpesvirus 1*, as well as the RNA-dependent RNA polymerase inhibitor remdesivir, and the RNA-dependent RNA polymerase activated mutagen molnupiravir, both clinically approved for the treatment of SARS-CoV-2 infection³. Remarkably, the human Viperin protein (Virus Inhibitory Protein, Endoplasmic Reticulum Associated, IFN-inducible), also known as RSAD2 (radical S-adenosyl methionine domain-containing 2), was recently shown *in vitro* to catalyze the formation of 3'-deoxy-3',4'-didehydro cytidine triphosphate (ddhCTP) via hydrogen atom abstraction and subsequent formal dehydration of cytidine triphosphate (CTP)⁴, S. Ghosh et al. 2020⁵ evidencing the presence of natural defense mechanisms against viruses. Specifically, the former compound, lacking a hydroxyl at position 3' of the degraded furanose ring system, was found to compete with cytidine triphosphate (CTP) for selective incorporation by various Flaviviral (Zika virus, West Nile virus, Dengue virus, Hepatitis-C-Virus (HCV)) RNA-dependent RNA polymerases into nascent strands of viral RNA, therein acting as an obligate chain terminator. Recently, the dephosphorylated form of ddhCTP, 3'-deoxy-3',4'-didehydro-cytidine (ddhC, 1) was detected in serum by liquid chromatography coupled to mass spectrometry (LC-MS) following SARS-CoV-2 infections. Mammalian viperin has also been shown to catalyze the formation of 3'-deoxy-3',4'-didehydro uridine triphosphate (ddhUTP) from uridine triphosphate (UTP), albeit having an *in vitro* substrate preference of CTP over UTP of approximately one thousand-fold under physiological conditions. Furthermore, homologues of the viperin enzyme have been discovered across all three domains of cellular life, with Fungal, Bacterial and Archaeal homologues additionally catalyzing the formation of ddhUTP and 3'-deoxy-3',4'-didehydro guanosine triphosphate (ddhGTP) from UTP and guanosine triphosphate (GTP) respectively. This suggests that these viral inhibitory mechanisms are ancient (the conservation of functional viperin homologues across all three domains and numerous bacterial phyla allowing a conservative estimate of >3.4 Bya) and finely refined by evolution. An understanding of the immunological processes related to these nucleotides is essential to evaluate them as diagnostic markers for viral infections, for example, in an intensive care unit environment where quick decision making is pivotal.

In addition to gauging the clinical and metabolic impact of SARS-CoV-2 infection, it is important to enhance the capabilities of quick viral testing to address the extent and rate of disease transmission. It was suggested that measurement of viral-induced metabolic phenoconversion provides a measure of the multiple systemic effects caused by the virus and allows insight into the pathological processes involved. Researchers have been investigating multiple acute pathological processes caused by SARS-CoV-2 infection in quest of novel biomarkers of infection, severity, recovery and long-term prognosis. A wide variety of biomarkers have been detected that help defining key aspects of the COVID-19 patient journey including amino acids, tryptophan metabolites⁰, lipids, lipoproteins, and other pro-inflammatory markers such as C-reactive proteins (CRP), lactic dehydrogenase (LDH) and ferritin. These metabolic perturbations are key to an understanding of the metabolic sequelae of SARS-CoV-2 infection and monitoring of its onset, but are not specific to the presence of a virus.

Thus, there is still a need for early markers that could accurately delineate the time frame of viral infections and enhance the ability to detect dormant viral infection with potential chronic consequences.

The technical problem underlying the present invention was therefore the provision of means and methods for complying with the aforementioned needs.

In the studies underlying the present invention, an extended network of deoxydidehydronucleosides (ddhNs) in the human bodyfluids such as urine and serum has been identified. The structures of eight metabolites were elucidated from urine using a combination of 1D and 2D NMR and high-resolution MS/MS: 3'-deoxy-3',4'-didehydro-cytidine (ddhC, 1), the dephosphorylated nucleoside derivative of ddhCTP and seven additional metabolites, previously unreported in human urine (and serum): 3',5'-dideoxy-3',4'-didehydrocytidine-5'-carboxylic acid (ddhC-5'CA, 2), 3'-deoxy-3',4'- didehydro-uridine (ddhU, 3), 3',5'-dideoxy-3',4'- didehydrocytidine-5'-homocysteine (ddhC-5'Hcy, 4), 3',5'-dideoxy-3',4'-didehydrouridine -5'-carboxylic acid (ddhU-5'CA, 5), 3',5'-dideoxy-3',4'-didehydrocytidine-5'-sulfoxide (ddhC-5'SO, 6), 3',5'-dideoxy-3',4'-didehydrocytidine-5'-thiomethyl (ddhC-5'MeS, 7) and 3',5'-dideoxy-3',4'- didehydrocytidine-5'-Methionine (ddhC-5'Met, 8), with additional hints that 6 and 8 are present as epimers in solution due to the chiral center of the sulfur. The three most abundant metabolites (1-3) were detected and quantified in urine and serum of participants at the onset of infection by SARS-CoV-2 using standard NMR and LC-MS analytics. Higher concentrations of 1-3 were found in more severe hospitalised patients compared to ambulant patients, with concentrations decreasing sharply a few days after onset. Furthermore, the NMR and MS data indicate the presence of multiple additional ddhNs. These unidentified compounds share the same spectroscopic signature and therefore must share the same 3',5'-dideoxy-3',4'-didehydro furanose moiety. This cornucopia of biologically active moieties suggests a synergistic response to viral infection, i.e., a virus inhibition pathway with extended biological function, thereby opening a new observational window into the innate immune response. Advantageously, the findings underlying the present invention allow for the development of a general diagnostic assay for acute viral infection with real-world clinical utility. Although the cohorts analyzed in this study were restricted to patients infected with SARS-CoV-2, it is expected that the newly identified biomarkers may prove useful in diagnosing alternative acute viral infections. A rapid diagnostic assay built around the disclosed biomarkers facilitated by the ease of urinary collection, would find ready application in intensive care units, allowing for the expeditious stratification of patients experiencing acute inflammatory distress. It is envisioned that an expedient assay allowing for the robust differentiation of viral infection from other sources of acute inflammation could provide tangible benefits in a clinical setting where resource and time allocation are essential to patient prognosis.

Accordingly, in a first aspect, the present invention is directed to a method for diagnosing a viral infection or a disease associated therewith in a subject comprising the steps of:
(a) determining in a sample of said subject the amount of at least one biomarker selected from the group of 3'(,5'-di)deoxy-3',4'-didehydro furanose derivatives of formula (I) 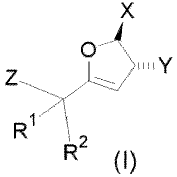 , wherein
   - R¹, R²: are each a hydrogen atom or together form a (double bonded) oxygen atom;
   - X: is selected from the group consisting of cytosine, uracil and thymine;
   - Y: is selected from the group consisting of hydrogen atom, hydroxyl group, -O-SO₃H group, and -O-PO₃H₂ group; and
   - Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂- CH(NH₂)-CO₂H group;
   with the exception of 3'-deoxy-3',4'-didehydro-cytidine (ddhC); and
(b) comparing the amount(s) of the at least one biomarkers determined in (a) with a reference, thereby diagnosing a viral infection.

The term "3'(,5'-di)deoxy-3',4'-didehydro furanose derivatives" comprises 3',5'-dideoxy-3',4'-didehydro furanose derivatives and 3'-deoxy-3',4'-didehydro furanose derivatives. A -S-(CH₂)₂-C(NH₂)-CO₂H group represents a homocysteine residue (Hcy), a -S⁺(CH₃)-(CH₂)₂-C(NH₂)-CO₂H group represents a methionine residue (Met⁺). The "I I I I" bond between the ring's C atom and Y indicates that said C atom is either S or R configured.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s).

The method as referred to in accordance with the present invention includes a method which essentially consists of the aforementioned steps or a method which includes further steps. However, it is to be understood that the method, in a preferred embodiment, is a method carried out ex vivo, i.e. not practiced on the human or animal body. The method, preferably, is assisted by automation. For example, sample processing or pre-treatment can be automated by robotics. Data processing and comparison is, preferably, assisted by suitable computer programs and databases, preferably as described elsewhere herein. Automation as described herein, preferably, allows using the method of the present invention in high-throughput approaches.

The term "diagnosing" as used herein refers to assessing whether a subject suffers from a viral infection or a disease associated therewith, or not. As will be understood by those skilled in the art, such an assessment, although preferred to be, may usually not be correct for 100% of the investigated subjects. The term, however, requires that a statistically significant portion of subjects can preferably be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in standard text books such as Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.1, 0.05, or 0.01. The term includes individual diagnosis of a viral infection or a disease associated therewith as well as continuous monitoring of a patient. Monitoring, i.e. diagnosing the presence or absence of a viral infection or a disease associated therewith or the symptoms accompanying it at various time points, includes monitoring of patients known to suffer from a viral infection or a disease associated therewith as well as monitoring of subjects known to be at risk of developing a viral infection or a disease associated therewith. Furthermore, monitoring can also be used to determine whether a patient is treated successfully or whether at least symptoms of a viral infection or a disease associated therewith can be ameliorated over time by a certain therapy.

The term "biomarker", as used herein, refers to a molecular species which serves as an indicator for a viral infection or a disease associated therewith as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. an analyte, will be the determined molecular species. Preferred chemical modifications may depend on the analytical method used and are described elsewhere herein. It is to be understood that in such a case, the analyte represents the actual metabolite and has the same potential as an indicator for the respective medical condition. Moreover, a biomarker according to the present invention is not necessarily corresponding to one molecular species. Rather, the biomarker may comprise stereoisomers or enantiomeres of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters.

A "metabolite", as used herein, refers to at least one molecule of a specific metabolite up to a plurality of molecules of the said specific metabolite. It is to be understood further that a group of metabolites means a plurality of chemically different molecules wherein for each metabolite at least one molecule up to a plurality of molecules may be present. A metabolite in accordance with the present invention may be from all classes of organic or inorganic chemical compounds including those being comprised by biological material such as organisms.

The term "determining the amount" as used herein refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, color, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said biomarker and its amount. Accordingly, the characteristic value, preferably, also comprises information relating to the abundance of the biomarker from which the characteristic value is derived. For example, a characteristic value of a biomarker may be a peak or peak pattern in a nuclear magnetic resonance spectrum (NMR spectrum) or a peak in a mass spectrum. For example, a peak in a mass spectrum contains characteristic information of the biomarker, as well as an intensity value being related to the abundance of the said biomarker (i.e. its amount) in the sample. Further, a peak or peak pattern in a NMR spectrum contains characteristic information of the biomarker (i.e. its amount) in the sample, which can be used for quantification, for example, by reliance on a reference of an electronic generated signal (ER-ETIC).

The amount of a biomarker comprised by a sample may be, preferably, determined quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case where the precise amount of a biomarker can or shall not be determined. In said case, it can preferably be determined whether the amount in which the biomarker is present is enlarged or diminished with respect to a second sample comprising said biomarker in a second amount. Quantitatively analysing a biomarker, thus, also includes what is sometimes referred to as semi-quantitative analysis of a biomarker. As is understood by the skilled person, the above applies to other measures of an amount of a compound, in particular a concentration, mutatis mutandis.

Preferably, determining comprises a sample pre-treatment step. Preferably, such pre-treatment may include treatments required to release or separate the compounds or to remove excessive material or waste. Suitable techniques comprise centrifugation, extraction, fractioning, ultrafiltration, protein precipitation followed by filtration and purification and/or enrichment of compounds. Preferably, other pre-treatments are carried out in order to provide the compounds in a form or concentration suitable for compound analysis. For example, if gas-chromatography coupled mass spectrometry is used in the method of the present invention, it may be required to derivatize the compounds prior to the said gas chromatography. Suitable and necessary pre-treatments depend on the means used for carrying out the method of the invention and are well known to the person skilled in the art. Samples pre-treated as described herein are also comprised by the term "sample" specified herein below.

Preferably, determining as used in the method of the present invention, includes using at least one compound separation step prior to the analysis step referred to before. Preferably, the compound separation step is a precipitation step, preferably causing precipitation of macromolecules comprised in the sample, in particular polypeptides. Preferably, said step comprises addition of from one to five volumes of a solution comprising an organic solvent to a liquid sample. Preferably, said solution comprising an organic solvent comprises methanol and or acetonitrile, more preferably is acetonitrile. Preferably, precipitated sample constituents are removed from the sample, preferably by centrifugation. Preferably, determination is performed on the supernatant of such precipitation. Also preferably, the compound separation step comprises a step yielding a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Appropriate techniques are disclosed in, e.g., Nissen 1995, Journal of Chromatography A, 703: 37-57, US 4,540,884 or US 5,397,894. More preferably, said mass spectrometry is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatized prior to gas chromatography. Suitable techniques for derivatization are well known in the art.

The term "reference" in connection with diagnostic methods is well known in the art. The reference in accordance with the present invention shall allow for the diagnosis of viral infection or disease associated therewith. A suitable reference may be established by the skilled person without further ado. The term reference, preferably, refers to values of characteristic features which can be correlated to a medical condition, i.e. the presence or absence of a viral infection, associated disease, diseases status or an effect referred to herein, or a calculated value or calculated values derived from said values. Preferably, the reference will be stored in a suitable data storage medium such as a database and are, thus, also available for future assessments. Preferably, the reference is a value determined and/or calculated from a subject or group of subjects known to suffer from viral infection or disease associated therewith or is a value determined and/or calculated from an apparently healthy subject or group thereof, i.e. a "reference amount". The reference to be applied may be an individual reference for each of the biomarkers to be determined in the method of the present invention. Based on the comparison of the amounts of the diagnostic biomarkers with the reference amounts, a diagnosis of viral infection or disease associated therewith, i.e. whether the subject as referred to herein suffers from viral infection or disease associated therewith, or not, is established. It is understood by the skilled person that a reference amount is not required to be an amount as determined in the determination step, but may also be a value derived therefrom by mathematical calculations well known to the skilled person. Preferably, a reference amount is a threshold value for a biomarker, preferably, a diagnostic biomarker, as referred to in connection with the present invention, whereby values found in a sample to be investigated which are higher than (or depending on the marker lower than) the threshold are indicative for the presence of viral infection or disease associated therewith while those being lower (or depending on the marker higher than) are indicative for the absence of viral infection or disease associated therewith.

The diagnostic algorithm, i.e. the specific set of calculation rules applied to obtain the diagnosis, may depend on the reference. If the reference amount is e.g. derived from a subject or group of subjects known to suffer from viral infection or disease associated therewith, the presence of viral infection or disease associated therewith is preferably indicated by amounts in the test sample which are essentially identical to the reference(s). If the reference amount is e.g. derived from an apparently healthy subject or group thereof, the presence of viral infection or disease associated therewith is preferably indicated by amounts of the diagnostic biomarkers in the test sample which are different from (e.g. increased ("up") or decreased ("down") as compared to the reference(s).

In accordance with the method, a reference amount (or reference amounts) is, preferably, a reference amount (or reference amounts) obtained from a sample from a subject or group of subjects known to suffer from viral infection or disease associated therewith. In such a case, a value for each of the diagnostic biomarkers found in the at least one test sample being essentially identical is indicative for the presence of the disease, i.e. of viral infection or disease associated therewith. Moreover, the reference amount, also preferably, could be from a subject or group of subjects known not to suffer from viral infection or disease associated therewith, preferably, an apparently healthy subject or group of subjects. In such a case, a value for each of the diagnostic biomarkers found in the test sample being altered with respect to the reference amount is indicative for the presence of the disease. Alternatively, a value for each of the diagnostic biomarkers found in the test sample being essentially identical with respect to the reference amount is indicative for the absence of the disease. The same applies mutatis mutandis for a calculated reference, most preferably the average or median, for the relative or absolute value of the diagnostic biomarkers in a population of individuals comprising the subject to be investigated. The absolute or relative values of the biomarkers of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

The value for a biomarker of the test sample and the reference amounts are essentially identical if the values for the characteristic features and, in the case of quantitative determination, the intensity values, or values derived therefrom, for the biomarker and the reference are essentially identical. Essentially identical means that the difference between two values is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value, preferably, the 50th, 60th, 70th, 80th, 90th or 95th percentile of the reference value. Statistical test for determining whether two amounts or values are essentially identical are well known in the art and are also described elsewhere herein. An observed difference for two values, on the other hand, shall be statistically significant. A difference in the relative or absolute value is, preferably, significant outside of the interval between 45th and 55th percentile, 40th and 60th percentile, 30th and 70th percentile, 20th and 80th percentile, 10th and 90th percentile, 5th and 95th percentile, 1st and 99th percentile of the reference value. In a preferred embodiment, the value for the characteristic feature can also be a calculated output such as score of a classification algorithm like "elastic net" as set forth elsewhere herein.

More preferably, the reference is derived from two subjects or groups of subjects known to differ in a property to be diagnosed, e.g. a subject or group of subjects known to suffer from viral infection or disease associated therewith and an apparently healthy subject or group thereof, e.g. as a reference score or as a cutoff value distinguishing between said two subjects or groups, as specified herein below. The skilled person understands that other subject groups which shall be distinguished can be used as well for establishing e.g. a cutoff value. Moreover, more than one cutoff value may be provided, e.g., two cutoff values may be determined, wherein the interval between the first and the second cutoff may define, preferably diagnosis of increased risk of suffering from a disease to be diagnosed, warranting, e.g., closer monitoring of the patient.

The term "comparing", preferably, refers to determining whether the determined values of the biomarkers or score are/is essentially identical to a reference or differs therefrom. Based on the comparison referred to above, a subject can be assessed to suffer from viral infection or disease associated therewith, or not. It is to be understood that the diagnostic algorithm may be adjusted to the reference or references to be applied. This is taken into account by the skilled person who can establish suitable reference values and/or diagnostic algorithms based on the diagnosis provided herein. The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

In some embodiments, the at least one biomarker is determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes, or aptamers. Specific antibodies, for instance, may be obtained using the biomarker as antigen by methods well known in the art. Antibodies as referred to herein include both polyclonal and monoclonal antibodies, as well as fragments thereof, such as Fv, Fab and F(ab)2 fragments that are capable of binding the antigen or hapten. The present invention also includes humanized hybrid antibodies wherein amino acid sequences of a non-human donor antibody exhibiting a desired antigenspecificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the biomarker are, preferably, enzymes which are involved in the metabolic conversion of the said biomarker. Said enzymes may either use the biomarker as a substrate or may convert a substrate into the biomarker. Moreover, said antibodies may be used as a basis to generate oligopeptides which specifically recognize the biomarker. These oligopeptides shall, for example, comprise the enzyme's binding domains or pockets for the said biomarker. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the biomarker may also be determined based on its capability to react with other compounds, i.e. by a specific chemical reaction. Further, the biomarker may be determined in a sample due to its capability to elicit a response in a biological read out system. The biological response shall be detected as read out indicating the presence and/or the amount of the biomarker comprised by the sample. The biological response may be, e.g., the induction of gene expression or a phenotypic response of a cell or an organism. In a preferred embodiment the determination of the least one biomarker is a quantitative process, e.g., allowing also the determination of the amount of the at least one biomarker in the sample.

More preferably, determining of the at least one biomarker comprises nuclear magnetic resonance spectroscopy (NMR) and/or mass spectrometry (MS). In some embodiments, the determining step comprises at least one further of the following techniques: magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado.

NMR as used herein encompasses all techniques which allow for the determination of NMR active nuclei (nuclei with a spin > 0) whose bulk magnetization gets aligned by external magnetic fields and rely on the detection/measurement of an electric signal/current after radiofrequency excitation of the NMR active nuclei's spins matching their respective resonance behaviour. The measured signal intensity is correlated to the quantity of a compound in a sample, i.e. a biomarker, to be determined in accordance with the present invention. Quantitative NMR (qNMR) is done using a reference substance or relying on the use as the reference of an electronically generated signal (ER-ETIC). Preferably, one or more NMR spectroscopy method(s) are used, in particular selected from ¹H NMR, ¹³C NMR, ¹H Total Correlated SpectroscopY (TOCSY), ¹H Nuclear Overhauser Enhancement and Exchange Spectroscopy (NOESY), ¹H¹H COrrelation SpectroscopY (COSY), ¹H¹H _{longrange}COSY, ¹H¹H TOCSY, ¹H¹H NOESY, ¹H¹³C Heteronuclear Single Quantum Coherence (HSQC), or ¹H¹³C Heteronuclear Multiple Bond Correlation (HMBC) or 1D ¹H (selective) versions of TOCSY, COSY, NOESY, HSQC and HMBC with ¹H detection. How to apply these techniques is well known to the person skilled in the art. Moreover, suitable devices are commercially available.

MS as used herein encompasses all techniques which allow for the determination of the molecular weight (i.e. the mass) or a mass variable corresponding to a compound, i.e. a biomarker, to be determined in accordance with the present invention. Preferably, one or more mass spectrometry methods are used, in particular selected from gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step.

In some preferred embodiments of the method for diagnosing a viral infection or a disease associated therewith, the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met). In some preferred embodiments of the method for diagnosing a viral infection or a disease associated therewith, the method further comprises determining the amount of 3'-deoxy-3',4'-didehydro-cytidine (ddhC) in said sample of the subject in step (a) and comparing the amount of ddhC to a reference in step (b).

The structures of the above-identified biomarkers and their related numbering used later herein are shown below:

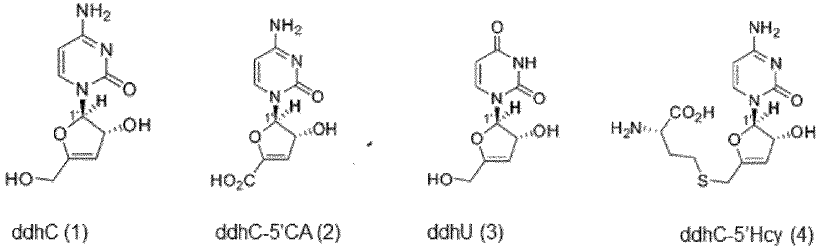

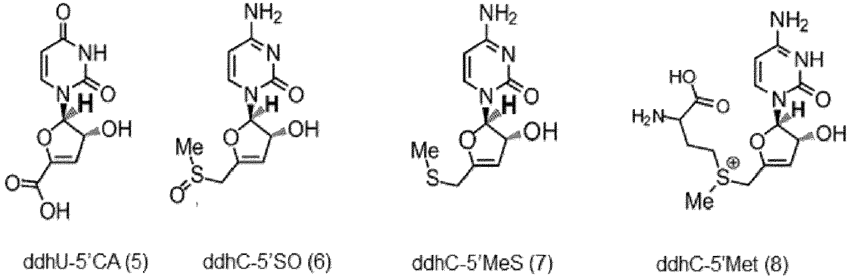

In some preferred embodiments of the method for diagnosing a viral infection or a disease associated therewith, the at least one biomarker determined in (a) and compared to a reference in (b) is selected from the group consisting of ddhC-5'CA, ddhU and ddhC-5'Hcy, preferably the at least one biomarker is ddhC-5'CA and/or ddhU. In some preferred embodiments of the method for diagnosing a viral infection or a disease associated therewith, determining the amount of the at least one biomarker in (a) is done by a method selected from the group consisting of mass spectrometry (MS), nuclear magnetic resonance (NMR) spectroscopy, liquid chromatography (LC), gas chromatography (GC) and a combination of two or more of these methods. In some preferred embodiments of the method for diagnosing a viral infection or a disease associated therewith, determining the amount of the at least one biomarker in (a) is done by NMR spectroscopy, preferably by ¹H-NMR spectroscopy, more preferably ¹H-NMR spectroscopy focused on at last one of the chemical shift regions δH in the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm.

In some preferred embodiments of the method for diagnosing a viral infection or a disease associated therewith, the sample of the subject is a tissue sample or a body fluid sample. In some preferred embodiments of the method for diagnosing a viral infection or a disease associated therewith, the body fluid sample is selected from the group consisting of: spinal fluid, lymph, blood, preferably whole blood, plasma or serum, gastrointestinal fluids, preferably saliva, gastric acid, pancreas fluid or bile, urine, and sweat. Techniques for obtaining the aforementioned different types of biological samples are well known in the art. For example, blood samples may be obtained by blood taking while tissue or organ samples are to be obtained, e.g., by biopsy. In some preferred embodiments of the method for diagnosing a viral infection or a disease associated therewith, the body fluid sample is a urine sample.

In an alternatively preferred embodiment, the invention is directed to a method for diagnosing a viral infection or a disease associated therewith in a subject comprising the steps of:
(A) determining in a sample of said subject the amount of 3'-deoxy-3',4'-didehydro-cytidine (ddhC); and
(B) comparing the amount of the ddhC determined in (A) with a reference, thereby diagnosing a viral infection;
wherein the determination in (a) is done by NMR spectroscopy and the sample of the subject is a urine sample of said subject.

In some preferred embodiments of the method for diagnosing a viral infection or a disease associated therewith the subject is a mammal, preferably a human. Preferably, the subject is a subject suspected to suffer from a viral infection or a disease associated therewith. Suspicion that a subject may suffer from a viral infection or a disease associated therewith, preferably, arises from at least one clinical symptom known to the skilled person to be associated with a viral infection or a disease associated therewith. Thus, preferably, the subject suspected to suffer from a viral infection or a disease associated therewith, preferably, is a subject having at least one clinical symptom of a viral infection or a disease associated therewith, such as fever, exhaustion, respiratory symptoms such as coughing, general body pain, sore throat, dyspnea.

In some preferred embodiments of the method for diagnosing a viral infection or a disease associated therewith the method is an *ex vivo* method. In some preferred embodiments of the method for diagnosing a viral infection or a disease associated therewith the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection, and SARS-CoV-2 infection, more preferably the viral infection is a SARS-CoV-2 infection. In some preferred embodiments of the method for diagnosing a viral infection or a disease associated therewith the associated disease is Corona Virus Disease 2019 (COVID-19). In some preferred embodiments of the method for diagnosing a viral infection or a disease associated therewith said reference is derived from the amount of the at least one biomarker from a subject or group of subjects known to suffer from the viral infection and/or the associated disease to be diagnosed. In some preferred embodiments of the method for diagnosing a viral infection or a disease associated therewith an amount for the at least one biomarker determined in step (a) which is essentially identical to the reference is indicative for a subject suffering from the said viral infection and/or the associated disease whereas an amount for the at least one biomarker determined in step (a) that differs from the reference is indicative for a subject not suffering from the viral infection and/or the associated disease. In some preferred embodiments of the method for diagnosing a viral infection or a disease associated therewith said reference is derived from the amount of the at least one biomarker from a subject or group of subjects known not to suffer from the viral infection and/or the associated disease to be diagnosed. In some preferred embodiments of the method for diagnosing a viral infection or a disease associated therewith an amount for the at least one biomarker determined in step (a) which is essentially identical to the reference is indicative for a subject not suffering from the said viral infection and/or the associated disease whereas an amount for the at least one biomarker determined in step (a) that differs from the reference is indicative for a subject suffering from the viral infection and/or the associated disease.

### 2^{nd} aspect - Method for identifying whether a subject is in need of a viral infection therapy

In a second aspect, the invention is directed to a method for identifying whether a subject is in need of a viral infection therapy comprising the steps of the method of any one of embodiments indicated above in the context of the first aspect and the further step of identifying a subject in need of a viral infection therapy if said subject is diagnosed to suffer from viral infection or from a disease associated therewith. In some preferred embodiments of the method for identifying whether a subject is in need of a viral infection therapy said viral infection therapy comprises drug treatment.

The phrase "in need of a viral infection therapy" as used herein means that the disease in the subject is in a status where therapeutic intervention is necessary or beneficial in order to ameliorate or treat a viral infection or the disease associated therewith or the symptoms associated therewith respectively. Accordingly, the findings of the studies underlying the present invention do not only allow diagnosing a viral infection or a disease associated therewith in a subject but also allow for identifying subjects which should be treated by a viral infection therapy or whose viral infection therapy needs adjustment. Once the subject has been identified, the method may further include a step of making recommendations for a therapy of viral infection or disease associated therewith. Preferably, the method further comprises the step of recommending a therapeutic or patient health management measure for the subject based on whether the subject is diagnosed to suffer from viral infection or disease associated therewith. The term "recommending", as used herein, refers to making suggestions for therapeutic measures and/or patient health management measures which are specifically applicable to the patient. Recommending does, preferably, not encompass the actual application of the recommended therapeutic or patient health management measure.

The term "therapeutic or patient health management measure" as used herein refers to therapeutic measures aiming to cure or ameliorate viral infection or disease associated therewith or aiming at preventing progression of the said diseases as well as patient health management measures such as monitoring including selection of monitoring measures and monitoring frequency and hospitalization. Preferably, the said therapeutic or patient health management measure is selected from the group consisting of: surgery, administration of anti-viral drugs, patient monitoring, active surveillance, and hospitalization. It will be understood that the method can also be applied to determine whether a subject will benefit from or is in need of a therapy against the aforementioned diseases. Such a method can be applied in therapeutic approaches like "active surveillance".

### 3^{rd} aspect - Means for use in diagnosing a viral infection or a disease associated therewith

A third aspect of the invention is related to a diagnostic means for use in diagnosing a viral infection or a disease associated therewith, said diagnostic means comprising at least one biomarker selected from the group of 3'(,5'-di)deoxy-3',4'-didehydro furanose derivatives of formula (I) 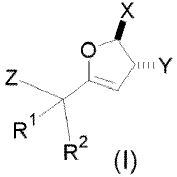 , wherein
- R¹, R²: are each a hydrogen atom or together form a (double bonded) oxygen atom;
- X: is selected from the group consisting of cytosine, uracil and thymine;
- Y: is selected from the group consisting of hydrogen atom, hydroxyl group, -O-SO₃H group, and -O-PO₃H₂ group; and
- Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;

with the exception of 3'-deoxy-3',4'-didehydro-cytidine (ddhC). In some preferred embodiments of the diagnostic means for use in diagnosing a viral infection or a disease associated therewith, the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'-didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met). In some preferred embodiments of the diagnostic means for use in diagnosing a viral infection or a disease associated therewith, said diagnostic means is further comprising 3'-deoxy-3',4'-didehydro-cytidine (ddhC) as a biomarker. In some preferred embodiments of the diagnostic means for use in diagnosing a viral infection or a disease associated therewith, the biomarker is selected from the group of consisting of ddhC-5'CA, ddhU and ddhC-5'Hcy. In some preferred embodiments of the diagnostic means for use in diagnosing a viral infection or a disease associated therewith, the biomarker is ddhC-5'CA and/or ddhU. In some preferred embodiments of the diagnostic means for use in diagnosing a viral infection or a disease associated therewith, diagnosing a viral infection comprises determining the amount of the diagnostic biomarker by a method selected from the group consisting of mass spectrometry (MS), nuclear magnetic resonance (NMR) spectroscopy, liquid chromatography (LC), gas chromatography (GC) and a combination of two or more of these methods. In some preferred embodiments of the diagnostic means for use in diagnosing a viral infection or a disease associated therewith, determining the amount of the diagnostic biomarker is done by NMR spectroscopy, preferably by ¹H-NMR spectroscopy, more preferably ¹H-NMR spectroscopy focused on at least one of the chemical shifts region δH in the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm. In some preferred embodiments of the diagnostic means for use in diagnosing a viral infection or a disease associated therewith, diagnosing a viral infection comprises determining the amount of the biomarker in a sample of the subject, which is preferably a tissue sample or a body fluid sample. In some preferred embodiments of the diagnostic means for use in diagnosing a viral infection or a disease associated therewith, the body fluid sample is selected from the group consisting of: spinal fluid, lymph, blood, preferably whole blood, plasma or serum, gastrointestinal fluids, preferably saliva, gastric acid, pancreas fluid or bile, urine, and sweat. In some preferred embodiments of the diagnostic means for use in diagnosing a viral infection or a disease associated therewith, the sample is a urine sample.

In an alternatively preferred embodiment, the invention relates to a diagnostic means comprising 3'-deoxy-3',4'-didehydro-cytidine (ddhC) for use in diagnosing a viral infection or a disease associated therewith, wherein the amount of the biomarker is determined done by NMR spectroscopy in a urine sample of a subject.

In some preferred embodiments of the diagnostic means for use in diagnosing a viral infection or a disease associated therewith, the subject is a mammal, preferably a human. In some preferred embodiments of the diagnostic means for use in diagnosing a viral infection or a disease associated therewith, the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection and SARS-CoV-2 infection, more preferably the viral infection isa SARS-CoV-2 infection. In some preferred embodiments of the diagnostic means for use in diagnosing a viral infection or a disease associated therewith, the said associated disease is Corona Virus Disease 2019 (COVID-19).

### 4^{th} aspect - Diagnostic use

In a fourth aspect, the invention relates to the use of at least one biomarker selected from the group of 3'(,5'-di)deoxy-3',4'-didehydro furanose derivatives of formula (I) 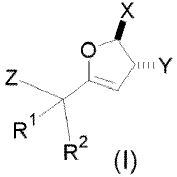 , wherein
- R¹, R²: are each a hydrogen atom or together form a (double bonded) oxygen atom;
- X: is selected from the group consisting of cytosine, uracil and thymine;
- Y: is selected from the group consisting of hydrogen atom, hydroxyl group, -O-SO₃H group, and -O-PO₃H₂ group; and
- Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
with the exception of 3'-deoxy-3',4'-didehydro-cytidine (ddhC) in a sample of a subject for diagnosing a viral infection or a disease associated therewith.

In some preferred embodiments of the use for diagnosing a viral infection or a disease associated therewith, the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met). In some preferred embodiments of the use for diagnosing a viral infection or a disease associated therewith, 3'-deoxy-3',4'-didehydro-cytidine (ddhC) in the said sample is used as further biomarker for diagnosing a viral infection. In some preferred embodiments of the use for diagnosing a viral infection or a disease associated therewith, the biomarker is selected from the group of consisting of ddhC-5'CA, ddhU and ddhC-5'Hcy. In some preferred embodiments of the use for diagnosing a viral infection or a disease associated therewith, the biomarker is ddhC-5'CA and/or ddhU. In some preferred embodiments of the use for diagnosing a viral infection or a disease associated therewith, diagnosing a viral infection comprises determining the amount of the diagnostic biomarker by a method selected from the group consisting of mass spectrometry (MS), nuclear magnetic resonance (NMR) spectroscopy, liquid chromatography (LC), gas chromatography (GC) and a combination of two or more of these methods. In some preferred embodiments of the use for diagnosing a viral infection or a disease associated therewith, determining the amount of the diagnostic biomarker is done by NMR spectroscopy, preferably by ¹H-NMR spectroscopy, more preferably ¹H-NMR spectroscopy focused on at least one of the chemical shift regions δH in the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm. In some preferred embodiments of the use for diagnosing a viral infection or a disease associated therewith, determining the amount of the diagnostic biomarker is done based on a sample of a subject, which is a tissue sample or a body fluid sample. In some preferred embodiments of the use for diagnosing a viral infection or a disease associated therewith, the body fluid sample is selected from the group consisting of: spinal fluid, lymph, blood, preferably whole blood, plasma or serum, gastrointestinal fluids, preferably saliva, gastric acid, pancreas fluid or bile, urine, and sweat. In some preferred embodiments of the use for diagnosing a viral infection or a disease associated therewith, the sample is a urine sample.

In an alternative embodiment, the invention is directed to a use of the biomarker 3'-deoxy-3',4'-didehydro-cytidine (ddhC), in a urine sample of a subject for diagnosing a viral infection or a disease associated therewith by NMR spectroscopy.

In some preferred embodiments of the use for diagnosing a viral infection or a disease associated therewith, the subject is a mammal, preferably a human. In some preferred embodiments of the use for diagnosing a viral infection or a disease associated therewith, the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection and SARS-CoV-2 infection, more preferably the viral infection is a SARS-CoV-2 infection. In some preferred embodiments of the use for diagnosing a viral infection or a disease associated therewith, the associated disease is Corona Virus Disease 2019 (COVID-19).

### 5^{th} aspect - Diagnostic device

A fifth aspect of the invention is related to a device for diagnosing a viral infection or a disease associated therewith in a sample of a subject comprising:
(i) an analyzing unit for the sample of the subject comprising a detector for the amounts of at least one biomarker selected from the group of 3'(,5'-di)deoxy-3',4'-didehydro furanose derivatives of formula (I) 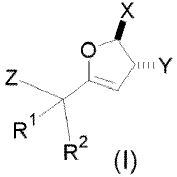 , wherein
   - R¹, R²: are each a hydrogen atom or together form a (double bonded) oxygen atom;
   - X: is selected from the group consisting of cytosine, uracil and thymine;
   - Y: is selected from the group consisting of hydrogen atom, hydroxyl group, -O-SO₃H group, and -O-PO₃H₂ group; and
   - Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
   with the exception of 3'-deoxy-3',4'-didehydro-cytidine (ddhC); said detector allowing for the determination of the amounts of the biomarkers of the said group of biomarkers in the sample; and operatively linked thereto,
(ii) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established.

In some preferred embodiments of the device, the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met). In some preferred embodiments of the device, the analyzing unit of (i) comprises a further detector for the amount of 3'-deoxy-3',4'-didehydro-cytidine (ddhC). In some preferred embodiments of the device, the analyzing unit of (i) comprises a detector for the amounts of at least one biomarker selected from the group consisting of ddhC-5'CA, ddhU, and ddhC-5'Hcy. In some preferred embodiments of the device, the analyzing unit of (i) comprises a detector for the amounts of ddhC-5'CA and/or ddhU. In some preferred embodiments of the device, the analyzing unit is an NMR analyzing unit and/or an MS analyzing unit.

A "device", as the term is used herein, comprises at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Preferably, the device includes an analyzing unit for the biomarkers and a computer or data processing device as an evaluation unit for processing the resulting data for the assessment and for establishing the output information. Preferably, the analyzing unit comprises at least one detector for at least the biomarkers of a group according to the present invention, said at least one detector determining the amounts of said biomarkers in said sample. Preferred devices are those which can be applied without the particular knowledge of a specialized clinician, e.g., electronic devices which merely require loading with a sample. The output information of the device, preferably, is a numerical value which allows drawing conclusions on the quality of the sample and, thus, is an aid for the reliability of a diagnosis or for troubleshooting. Preferred references to be used in accordance with the device of the present invention are values for the biomarkers analyzed or values derived therefrom as specified above. Preferably, the device further comprises, operatively linked to the evaluation unit, at least one input unit for inputting the amount of a marker and/or an analyzing unit for the sample of the subject comprising a detector for the amount of a marker in said sample. Preferably, the device further comprises an input unit adapted to receive input data.

The units of the device, also preferably, can be implemented into a system comprising several devices which are operatively linked to each other. Depending on the units to be used for the system of the present invention, said means may be functionally linked by connecting each means with the other by means which allow data transport in between said means, e.g., glass fiber cables, and other cables for high throughput data transport. Nevertheless, wireless data transfer between the means is also envisaged by the present invention, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise NMR devices and/or mass spectrometry devices. The separation and determination means are, preferably, coupled to each other. Further comprised shall be means for comparing and/or analyzing the results obtained from the means for determination of biomarkers. The means for comparing and/or analyzing the results may comprise at least one databases and an implemented computer program for comparison of the values measured with corresponding references. Preferred embodiments of the aforementioned systems and devices are also described in detail below.

Furthermore, the present invention relates to a data collection comprising characteristic values of at least the markers of at least one panel disclosed herein, being indicative for a subject suffering from a viral infection or a disease associated therewith, or not.

The term "data collection" refers to a collection of data which may be physically and/or logically grouped together. Accordingly, the data collection may be implemented in a single data storage medium or in physically separated data storage media being operatively linked to each other. Preferably, the data collection is implemented by means of a database. Thus, a database as used herein comprises the data collection on a suitable storage medium. Moreover, the database, preferably, further comprises a database management system. The database management system is, preferably, a network-based, hierarchical or object-oriented database management system. Furthermore, the database may be a federal or integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for a viral infection or a disease associated therewith as set forth above (e.g. a query search). Thus, if an identical or similar data set can be identified in the data collection, the test data set will be associated with the presence of disease, or not. Consequently, the information obtained from the data collection can be used, e.g., as a reference for the methods of the present invention described above. More preferably, the data collection comprises characteristic values of all diagnostic biomarkers comprised by any one of the groups recited above.

In light of the foregoing, the present invention encompasses a data storage medium comprising the aforementioned data collection.

The term "data storage medium" as used herein encompasses data storage media which are based on single physical entities such as a CD, a CD-ROM, a hard disk, optical storage media, or a diskette. Moreover, the term further includes data storage media consisting of physically separated entities which are operatively linked to each other in a manner as to provide the aforementioned data collection, preferably, in a suitable way for a query search.

### 6^{th} aspect - Calibration sample

A sixth aspect of the invention relates to a calibration sample comprising at least one biomarker selected from the group of 3'(,5'-di)deoxy-3',4'-didehydro furanose derivatives of formula (I) 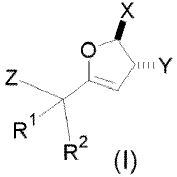 , wherein
- R¹, R²: are each a hydrogen atom or together form a (double bonded) oxygen atom;
- X: is selected from the group consisting of cytosine, uracil and thymine;
- Y: is selected from the group consisting of hydrogen atom, hydroxyl group, -O-SO₃H group, and -O-PO₃H₂ group; and
- Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
with the exception of 3'-deoxy-3',4'-didehydro-cytidine (ddhC) in dissolved form. In some preferred embodiments of the calibration sample, the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met). In some preferred embodiments the calibration sample comprises 3'-deoxy-3',4'-didehydro-cytidine (ddhC). In some preferred embodiments of the calibration sample, the at least one biomarker is selected from the group consisting of ddhC-5'CA, ddhU, and ddhC-5'Hcy. In some preferred embodiments of the calibration sample, the at least one biomarker is ddhC-5'CA and/or ddhU.

### 7^{th} aspect - Kit

In seventh aspect, the invention is directed to a kit for identifying a subject suffering from a viral infection or from a disease associated therewith, the kit comprising
(i) a reference sample of at least one biomarker selected from the group of 3'(,5'-di)deoxy-3',4'-didehydro furanose derivatives of formula (I) 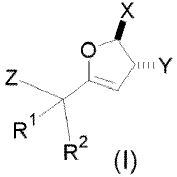 , wherein
   - R¹, R²: are each a hydrogen atom or together form a (double bonded) oxygen atom;
   - X: is selected from the group consisting of cytosine, uracil and thymine;
   - Y: is selected from the group consisting of hydrogen atom, hydroxyl group, -O-SO₃H group, and -O-PO₃H₂ group; and
   - Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
   and/or
(ii) at least one binding agent capable of binding at least one biomarker selected from the group of 3'(,5'-di)deoxy-3',4'-didehydro furanose derivatives of formula (I).

In some preferred embodiments of the kit, w 3'-deoxy-3',4'-didehydro-cytidine (ddhC) is excluded from the group of biomarkers.

In some preferred embodiments of the kit, the least one biomarker of (i) is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met); and/or wherein the at least one binding agent capable of binding at least one biomarker of (ii) is at least one binding agent capable of binding at least one biomarker selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met). In some preferred embodiments, the kit further comprises a reference sample of 3'-deoxy-3',4'-didehydro-cytidine (ddhC) and/or at least one binding agent capable of binding 3'-deoxy-3',4'-didehydro-cytidine (ddhC). In some preferred embodiments of the kit said binding agent is an antibody or aptamer. In some preferred embodiments of the kit said kit further comprises one or more reagents for sample preparation and/or one or more reagents for sample extraction.

The present invention is further illustrated by the following embodiments and combinations of embodiments as indicated by the respective dependencies and back-references. In particular, it is noted that in each instance where a range of embodiments is mentioned, for example in the context of a term such as "The ... of any of embodiments 1 to 4", every embodiment in this range is meant to be explicitly disclosed for the skilled person, i.e. the wording of this term is to be understood by the skilled person as being synonymous to "The ... of any of embodiments 1, 2, 3, and 4". The definitions and explanations of the terms made before apply mutatis mutandis for the following embodiments.

Embodiment 1: A method for diagnosing a viral infection or a disease associated therewith in a subject comprising the steps of:
(a) determining in a sample of said subject the amount of at least one biomarker selected from the group of 3'(,5'-di)deoxy-3',4'-didehydro furanose derivatives of formula (I) 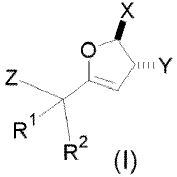 , wherein
   - R¹, R²: are each a hydrogen atom or together form a (double bonded) oxygen atom;
   - X: is selected from the group consisting of cytosine, uracil and thymine;
   - Y: is selected from the group consisting of hydrogen atom, hydroxyl group, -O-SO₃H group, and -O-PO₃H₂ group; and
   - Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂- CH(NH₂)-CO₂H group;
   with the exception of 3'-deoxy-3',4'-didehydro-cytidine (ddhC); and
(b) comparing the amount(s) of the at least one biomarkers determined in (a) with a reference, thereby diagnosing a viral infection.

Embodiment 2: The method of embodiment 1, wherein the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-methionine (ddhC-5'Met).

Embodiment 3: The method of embodiment 2, wherein the method further comprises determining the amount of 3'-deoxy-3',4'-didehydro-cytidine (ddhC) in said sample of the subject in step (a) and comparing the amount of ddhC to a reference in step (b).

Embodiment 4: The method of any one of embodiments 1 to 3, wherein the at least one biomarker determined in (a) and compared to a reference in (b) is selected from the group consisting of ddhC-5'CA, ddhU and ddhC-5'Hcy, preferably the at least one biomarker is ddhC-5'CA and/or ddhU.

Embodiment 5: The method of embodiments 1 to 4, wherein determining the amount of the at least one biomarker in (a) is done by a method selected from the group consisting of mass spectrometry (MS), nuclear magnetic resonance (NMR) spectroscopy, liquid chromatography (LC), gas chromatography (GC) and a combination of two or more of these methods.

Embodiment 6: The method of embodiment 5, wherein determining the amount of the at least one biomarker in (a) is done by NMR spectroscopy, preferably by ¹H-NMR spectroscopy, more preferably ¹H-NMR spectroscopy focused on at last one of the chemical shift regions δHin the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm.

Embodiment 7: The method of any one of embodiments 1 to 6, wherein the sample of the subject is a tissue sample or a body fluid sample.

Embodiment 8: The method of embodiment 7, wherein the body fluid sample is selected from the group consisting of: spinal fluid, lymph, blood, preferably whole blood, plasma or serum, gastrointestinal fluids, preferably saliva, gastric acid, pancreas fluid or bile, urine, and sweat.

Embodiment 9: The method for diagnosing a viral infection of any embodiment 8, wherein the body fluid sample is a urine sample.

Embodiment 10: A method for diagnosing a viral infection or a disease associated therewith in a subject comprising the steps of:
(A) determining in a sample of said subject the amount of 3'-deoxy-3',4'-didehydro-cytidine (ddhC); and
(B) comparing the amount of the ddhC determined in (A) with a reference, thereby diagnosing a viral infection;
wherein the determination in (a) is done by NMR spectroscopy and the sample of the subject is a urine sample of said subject.

Embodiment 11: The method of any one of embodiments 1 to 10, wherein the subject is a mammal, preferably a human.

Embodiment 12: The method of any one of embodiments 1 to 11, wherein the method is an *ex vivo* method.

Embodiment 13: The method of any one of embodiments 1 to 12, wherein the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection, and SARS-CoV-2 infection, more preferably the viral infection is a SARS-CoV-2 infection.

Embodiment 14: The method of any one of embodiments 1 to 13, wherein the associated disease is Corona Virus Disease 2019 (COVID-19).

Embodiment 15: The method of any one of embodiments 1 to 14, wherein said reference is derived from the amount of the at least one biomarker from a subject or group of subjects known to suffer from the viral infection and/or the associated disease to be diagnosed.

Embodiment 16: The method of embodiment 15, wherein an amount for the at least one biomarker determined in step (a) which is essentially identical to the reference is indicative for a subject suffering from the said viral infection and/or the associated disease whereas an amount for the at least one biomarker determined in step (a) that differs from the reference is indicative for a subject not suffering from the viral infection and/or the associated disease.

Embodiment 17: The method of any one of embodiments 1 to 16, wherein said reference is derived from the amount of the at least one biomarker from a subject or group of subjects known not to suffer from the viral infection and/or the associated disease to be diagnosed.

Embodiment 18: The method of embodiment 17, wherein an amount for the at least one biomarker determined in step (a) which is essentially identical to the reference is indicative for a subject not suffering from the said viral infection and/or the associated disease whereas an amount for the at least one biomarker determined in step (a) that differs from the reference is indicative for a subject suffering from the viral infection and/or the associated disease.

Embodiment 19: A method for identifying whether a subject is in need of a viral infection therapy comprising the steps of the method of any one of embodiments 1 to 18 and the further step of identifying a subject in need of a viral infection therapy if said subject is diagnosed to suffer from viral infection or a disease associated therewith.

Embodiment 20: The method of embodiment 19, wherein said viral infection therapy comprises drug treatment.

Embodiment 21: A diagnostic means for use in diagnosing a viral infection or a disease associated therewith, said diagnostic means comprising at least one biomarker selected from the group of 3'(,5'-di)deoxy-3',4'-didehydro furanose derivatives of formula (I) 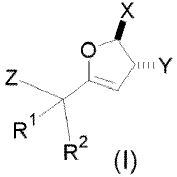 , wherein
- R¹, R²: are each a hydrogen atom or together form a (double bonded) oxygen atom;
- X: is selected from the group consisting of cytosine, uracil and thymine;
- Y: is selected from the group consisting of hydrogen atom, hydroxyl group, -O-SO₃H group, and -O-PO₃H₂ group; and
- Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
with the exception of 3'-deoxy-3',4'-didehydro-cytidine (ddhC).

Embodiment 22: The diagnostic means for use of embodiment 21, wherein the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'-didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'-didehydrocytidine-5'-Methionine (ddhC-5'Met).

Embodiment 23: The diagnostic means for use of embodiment 22, wherein said diagnostic means is further comprising 3'-deoxy-3',4'-didehydro-cytidine (ddhC) as a biomarker.

Embodiment 24: The diagnostic means for use of any one of embodiments 21 to 23, wherein the biomarker is selected from the group of consisting of ddhC-5'CA, ddhU and ddhC-5'Hcy.

Embodiment 25: The diagnostic means for use of any one of embodiments 21 to 24, wherein the biomarker is ddhC-5'CA and/or ddhU.

Embodiment 26: The diagnostic means for use of any one of embodiments 21 to 25, wherein diagnosing a viral infection comprises determining the amount of the diagnostic biomarker by a method selected from the group consisting of mass spectrometry (MS), nuclear magnetic resonance (NMR) spectroscopy, liquid chromatography (LC), gas chromatography (GC) and a combination of two or more of these methods.

Embodiment 27: The diagnostic means for use of embodiment 26, wherein determining the amount of the diagnostic biomarker is done by NMR spectroscopy, preferably by ¹H-NMR spectroscopy, more preferably ¹H-NMR spectroscopy focused on at least one of the chemical shifts region δHin the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm.

Embodiment 28: The diagnostic means for use of any one of embodiments 21 to 27, wherein diagnosing a viral infection comprises determining the amount of the biomarker in a sample of the subject, which is preferably a tissue sample or a body fluid sample.

Embodiment 29: The diagnostic means for use of embodiment 28, wherein the body fluid sample is selected from the group consisting of: spinal fluid, lymph, blood, preferably whole blood, plasma or serum, gastrointestinal fluids, preferably saliva, gastric acid, pancreas fluid or bile, urine, and sweat.

Embodiment 30: The diagnostic means for use of embodiment 29, wherein the sample is a urine sample.

Embodiment 31: A diagnostic means comprising 3'-deoxy-3',4'-didehydro-cytidine (ddhC) for use in diagnosing a viral infection or a disease associated therewith, wherein the amount of the biomarker is determined done by NMR spectroscopy in a urine sample of a subject.

Embodiment 32: The diagnostic means for use of any one of embodiments 21 to 31, wherein the subject is a mammal, preferably a human.

Embodiment 33: The diagnostic means for use of any one of embodiments 21 to 32, wherein the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection and SARS-CoV-2 infection, more preferably the viral infection isa SARS-CoV-2 infection.

Embodiment 34: The diagnostic means of any one of embodiments 21 to 32, wherein the said associated disease is Corona Virus Disease 2019 (COVID-19).

Embodiment 35: Use of at least one biomarker selected from the group of 3'(,5'-di)deoxy-3',4'-didehydro furanose derivatives of formula (I) 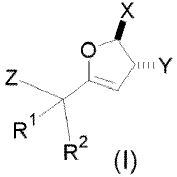 , wherein
- R¹, R²: are each a hydrogen atom or together form a (double bonded) oxygen atom;
- X: is selected from the group consisting of cytosine, uracil and thymine;
- Y: is selected from the group consisting of hydrogen atom, hydroxyl group, -O-SO₃H group, and -O-PO₃H₂ group; and
- Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
with the exception of 3'-deoxy-3',4'-didehydro-cytidine (ddhC) in a sample of a subject for diagnosing a viral infection or a disease associated therewith.

Embodiment 36: The use of embodiment 35, wherein the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met).

Embodiment 37: The use of embodiment 36, wherein 3'-deoxy-3',4'-didehydro-cytidine (ddhC) in the said sample is used as further biomarker for diagnosing a viral infection.

Embodiment 38: The use of any one of embodiments 35 to 37, wherein the biomarker is selected from the group of consisting of ddhC-5'CA, ddhU and ddhC-5'Hcy.

Embodiment 39: The use of any one of embodiments 35 to 38, wherein the biomarker is ddhC-5'CA and/or ddhU.

Embodiment 40: The use of any one of embodiments 35 to 39, wherein diagnosing a viral infection comprises determining the amount of the diagnostic biomarker by a method selected from the group consisting of mass spectrometry (MS), nuclear magnetic resonance (NMR) spectroscopy, liquid chromatography (LC), gas chromatography (GC) and a combination of two or more of these methods.

Embodiment 41: The use of embodiment 40, wherein determining the amount of the diagnostic biomarker is done by NMR spectroscopy, preferably by ¹H-NMR spectroscopy, more preferably ¹H-NMR spectroscopy focused on at least one of the chemical shift regions δH in the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm.

Embodiment 42: The use of any one of embodiments 35 to 41, wherein determining the amount of the diagnostic biomarker is done based on a sample of a subject, which is a tissue sample or a body fluid sample.

Embodiment 43: The use of any one of embodiments 35 to 42, wherein the body fluid sample is selected from the group consisting of: spinal fluid, lymph, blood, preferably whole blood, plasma or serum, gastrointestinal fluids, preferably saliva, gastric acid, pancreas fluid or bile, urine, and sweat.

Embodiment 44 The use of embodiment 43, wherein the sample is a urine sample.

Embodiment 45: Use of the biomarker 3'-deoxy-3',4'-didehydro-cytidine (ddhC), in a urine sample of a subject for diagnosing a viral infection or a disease associated therewith by NMR spectroscopy.

Embodiment 46: The use of embodiment 45, wherein the subject is a mammal, preferably a human.

Embodiment 47: The use of embodiment 45 or 46, wherein the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection and SARS-CoV-2 infection, more preferably the viral infection is a SARS-CoV-2 infection.

Embodiment 48: The use of any one of embodiments 45 to 47, wherein the associated disease is Corona Virus Disease 2019 (COVID-19).

Embodiment 49: The use of any one of embodiments 35 to 48, wherein the at least one biomarker is selected from the group comprising 3'-deoxy-3',4'-didehydro-cytidine (ddhC), 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydro-uridine -5'-carboxylic acid (ddhU-5'CA), 3',5'-dideoxy-3',4'-didehydrocytidine-5'-thiomethyl-sulfoxide (ddhC-5'SO), and 3',5'-dideoxy-3',4'-didehydrocytidine-5'-thiomethyl (ddhC-5'MeS), the viral infection is preferably a SARS-CoV-2 infection, or a disease associated therewith, preferably Corona Virus Disease 2019 (COVID-19), diagnosing is done by NMR spectroscopy in a urine sample of a subject, wherein the subject is preferably a mammal, more preferably a human.

Embodiment 50: A device for diagnosing a viral infection or a disease associated therewith in a sample of a subject comprising:
(i) an analyzing unit for the sample of the subject comprising a detector for the amounts of at least one biomarker selected from the group of 3'(,5'-di)deoxy-3',4'-didehydro furanose derivatives of formula (I) 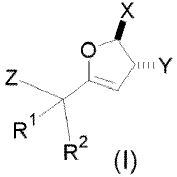 , wherein
   - R¹, R²: are each a hydrogen atom or together form a (double bonded) oxygen atom;
   - X: is selected from the group consisting of cytosine, uracil and thymine;
   - Y: is selected from the group consisting of hydrogen atom, hydroxyl group, -O-SO₃H group, and -O-PO₃H₂ group; and
   - Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
   with the exception of 3'-deoxy-3',4'-didehydro-cytidine (ddhC); said detector allowing for the determination of the amounts of the biomarkers of the said group of biomarkers in the sample; and operatively linked thereto,
(ii) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established.

Embodiment 51: The device of embodiment 50, wherein the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met).

Embodiment 52: The device of embodiment 51, wherein the analyzing unit of (i) comprises a further detector for the amount of 3'-deoxy-3',4'-didehydro-cytidine (ddhC).

Embodiment 53: The device of any one of embodiments 50 to 52, wherein the analyzing unit of (i) comprises a detector for the amounts of at least one biomarker selected from the group consisting of ddhC-5'CA, ddhU, and ddhC-5'Hcy.

Embodiment 54: The device of any one of embodiments 50 to 53, wherein the analyzing unit of (i) comprises a detector for the amounts of ddhC-5'CA and/or ddhU.

Embodiment 55: The device of any one of embodiments 50 to 54, wherein the analyzing unit is an NMR analyzing unit and/or an MS analyzing unit.

Embodiment 56: A calibration sample comprising at least one biomarker selected from the group of 3'(,5'-di)deoxy-3',4'-didehydro furanose derivatives of formula (I) with the exception of 3'-deoxy-3',4'-didehydro-cytidine (ddhC) in dissolved form.

Embodiment 57: The calibration sample of embodiment 56, wherein the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-Methionine (ddhC-5'Met).

Embodiment 58: The calibration sample of embodiment 57, further comprising 3'-deoxy-3',4'-didehydro-cytidine (ddhC).

Embodiment 59: The calibration sample of any one of embodiments 56 to 58, wherein the at least one biomarker is selected from the group consisting of ddhC-5'CA, ddhU, and ddhC-5'Hcy.

Embodiment 60: The calibration sample of any one of embodiments 56 to 59, wherein the at least one biomarker is ddhC-5'CA and/or ddhU.

Embodiment 61: A kit for identifying a subject suffering from a viral infection or a disease associated therewith, the kit comprising
(i) a reference sample of at least one biomarker selected from the group of 3'(,5'-di)deoxy-3',4'-didehydro furanose derivatives of formula (I) 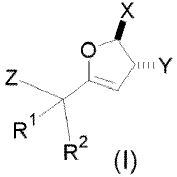 , wherein
   - R¹, R²: are each a hydrogen atom or together form a (double bonded) oxygen atom;
   - X: is selected from the group consisting of cytosine, uracil and thymine;
   - Y: is selected from the group consisting of hydrogen atom, hydroxyl group, -O-SO₃H group, and -O-PO₃H₂ group; and
   - Z: is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂-CH(NH₂)-CO₂H group;
   , and/or
(ii) at least one binding agent capable of binding at least one biomarker selected from the group of 3'(,5'-di)deoxy-3',4'-didehydro furanose derivatives of formula (I).

Embodiment 62: The kit of embodiment 61, wherein 3'-deoxy-3',4'-didehydro-cytidine (ddhC) is excluded from the group of biomarkers.

Embodiment 63: The kit of embodiment 61 or 62, wherein the least one biomarker of (i) is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-methionine (ddhC-5'Met); and/or wherein the at least one binding agent capable of binding at least one biomarker of (ii) is at least one binding agent capable of binding at least one biomarker selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydrocytidine-5'-thiomethyl (ddhC-5'MeS) and 3',5'-dideoxy-3',4'- didehydrocytidine-5'-methionine (ddhC-5'Met).

Embodiment 64: The kit of embodiment 63, further comprising a reference sample of 3'-deoxy-3',4'-didehydro-cytidine (ddhC) and/or at least one binding agent capable of binding 3'-deoxy-3',4'-didehydro-cytidine (ddhC).

Embodiment 65: The kit of any one of embodiments 62 to 64, wherein said binding agent is an antibody or aptamer.

Embodiment 66: The kit of any one of embodiments 62 to 65, wherein said kit further comprises one or more reagents for sample preparation and/or one or more reagents for sample extraction.

All references cited throughout this specification are herewith incorporated by reference with respect to the specifically mentioned disclosure content as well as in their entireties.

### FIGURES

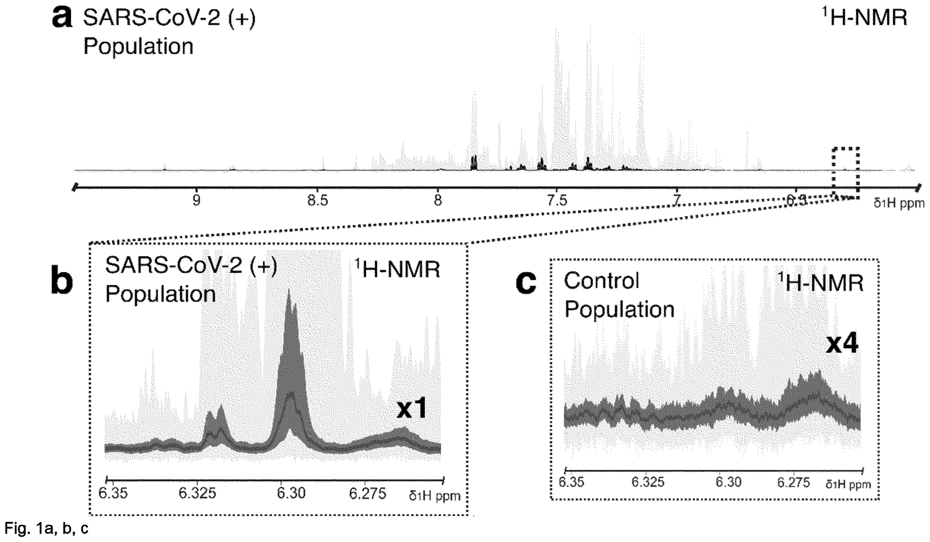

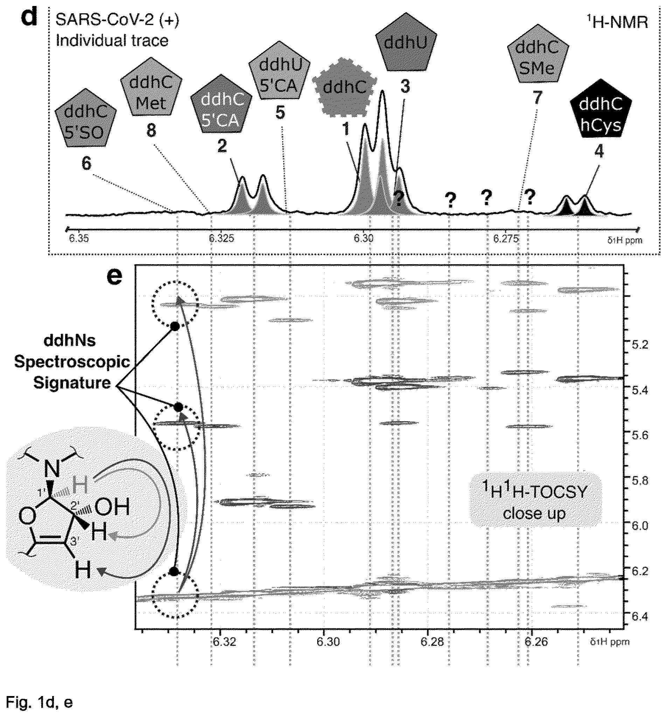

Fig. 1: ¹H NMR (600 AND 800 MHZ) metabolic fingerprints of urine from patients with SARS-CoV-2 infections: a) Statistical projection of 273 proton NMR spectra from the SARS-CoV-2 (+) cohort focussing on the high frequency part of the NMR spectrum. The median NMR spectrum (blue line) was shown with projected upper (Q3) and lower (Q1) quartiles (defined by dark gray area) and the maximum and minimum spectral intensities (light gray). b) Expanded spectral window of the SARS-CoV-2 (+) cohort focusing on the region δ_{H} 6.24-6.35 ppm. Participants from the infected cohort showed multiple patterns from δ_{H} 6.26-6.32 ppm. c) Comparative spectral window of the projected NMR spectra from the control cohort focusing on the region δ_{H} 6.25-6.35 ppm. No distinct peaks could be found and the spectral region was dominated by noise. All urine NMR spectra were PQN normalized. d) Exemplary proton urine NMR spectrum of a SARS-CoV-2 (+) participant depicting the region δ_{H} 6.25-6.35 ppm. Four doublets stemming from four different molecular species could be discerned (two of the doublets were overlapping; the individual lines were shown as colored lorentzian line shapes), which could be assigned to the anomeric protons of the four nucleoside species, 3'-deoxy-3',4'-didehydro-cytidine (ddhC, **1),** 3',5'-dideoxy-3',4'-didehydrocytidine-5'-carboxylic acid (ddhC-5'CA, 2), 3'-deoxy-3',4'-didehydro-uridine (ddhU, **3)** and 3',5'-dideoxy-3',4'-didehydrocytidine-5'-homocysteine (ddhC-5'Hcy, **4).** The additional metabolites 3',5'-dideoxy-3',4'-didehydrouridine-5'-carboxylic acid (ddhU-5'CA, 5), 3',5'-dideoxy-3',4'-didehydrocytidine-5'-thiomethylsulfoxide (ddhC-5'SO, **6),** 3',5'-dideoxy-3',4'-didehydrocytidine-5'-thiomethyl (ddhC-5'MeS, **7),** and 3',5'-dideoxy-3',4'- didehydrocytidine-5'-Methionine (ddhC-5'Met, **8)** were detected in fractionated urine and dotted lines indicate their anomeric proton chemical shift in urine; **5, 6, 7** and **8** were usually below the limit of detection in standard urine ¹H NMR acquisition (~32 scans, 600 MHz). e) ¹H¹H-TOCSY spectrum of the nucleoside "fingerprint" region for the same subject linking the anomeric proton H-1' (blue) to the full ddhN characteristic allylic H-2' (purple) and vinylic H-3' (green) positions of the dehydrated furanose spin system. In total, 12 ddhN spin systems could be detected.

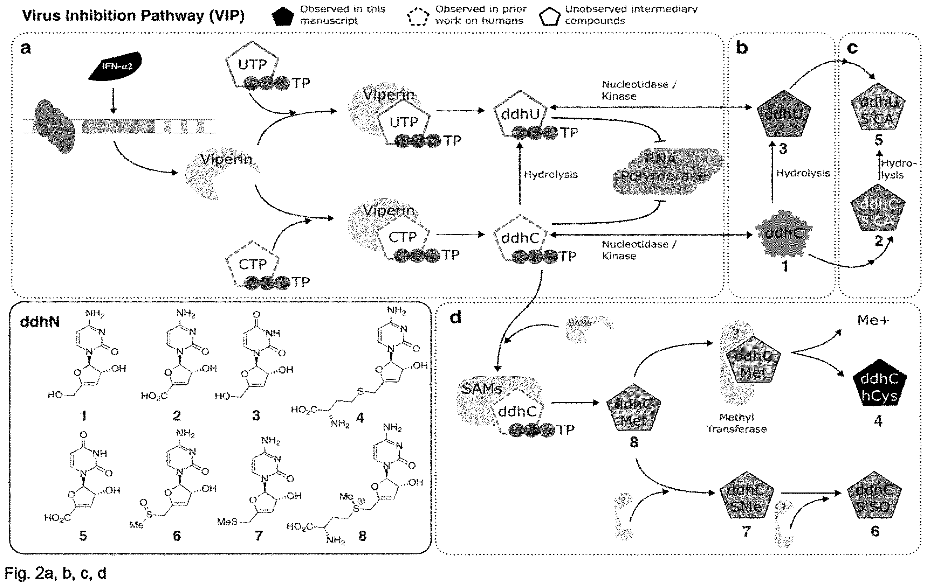

**Fig. 2****:** Biosynthetic network leading to the formation of ddhN species. a) Viperin pathway. b) Nucleotidase branch. c) Oxidative Branch. d) Methionine conjugative branch. Fig. legend: pentagons filled with different shades of gray/black: ddhN metabolites observed in this work, Dashed outlines: metabolites observed or postulated to exist in humans in prior work, Solid outlines without gray-fill: Unobserved compounds postulated to exist in humans based on this work. Chemical structures of detected ddhN species: ddhC **(1),** ddhC-5'CA **(2),** ddhU **(3),** ddhC-5'Hcy **(4),** ddhU-5'CA **(5),** ddhC-5'SO **(6),** ddhC-5'MeS **(7)** and ddhC-5'Met **(8).**

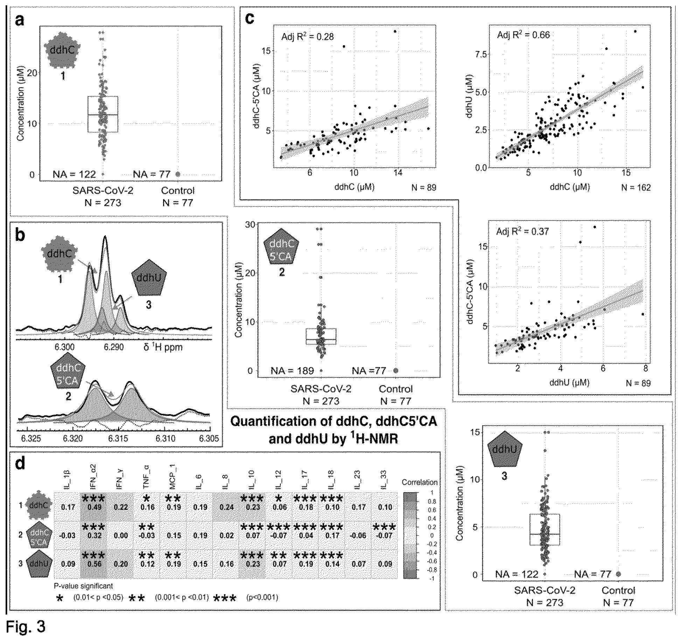

**Fig. 3****:** Quantification of ddhN species 1-3 in urine and serum, a) Box plots for compounds 1-3 concentration comparison between the SARS-CoV-2 infection during the acute phase and uninfected control cohort measured by NMR spectroscopy. b) Lorentzian fitting models and statistical analyses comparing the concentration of compounds 1-3 in SARS-CoV-2 (+) human urine quantified by NMR with exemplary Lorentzian fits for a spectrum of compounds 1-3 for two participants illustrated. c) Linear regression plots of compound 1-3 concentrations measured in urine by NMR. The concentration for all three compounds was given per mM creatinine. All three metabolites were below the limit of detection for all control samples and were not observable following visual inspection of the acquired spectra.

In the Figures, the compounds are abbreviated as follows:

| | |
|---|---|
| dhC | ddhC |
| ddhC-5'CA | ddhC 5'CA |
| ddhU | ddhU |
| ddhC-5'Hcy | ddhChCys |
| ddhU-5'CA | ddhU 5'CA |
| ddhC-5'SO | ddhC 5'SO |
| ddhC-5'MeS | ddhC SMe |
| ddhC-5'Met | ddhC Met |

### EXAMPLES

The following Examples shall merely illustrate the invention. They shall not be construed whatsoever as limiting its scope.

### Example 1: General methods and materials

### Chemicals and consumables

Phosphate buffer (1.5 M KH₂PO₄, 2 mM NaN₃, 0.1% sodium trimethylsilyl propionate-[2,2,3,3⁻²*H*₄] (TSP) in H₂O/D₂O 4:1, pH 7.4 ±0.1), and all NMR tubes (5 mm outer diameter SampleJet^{™} NMR tubes and regular 7 inch, 5 mm NMR tubes) with the corresponding sealing caps were purchased from Bruker A.G. Rheinstetten).

Organic solvents were all Optima-LCMS grade and purchased from Thermo Fischer Scientific (Malaga, WA, Australia). LC-MS grade water was generated from a Milli-Q IQ 7000 (Merck Millipore).

### Cohorts

### Heidelberg

Serum and urine samples of the COVID-19 cohort originate from an ambulatory treated- patient collective in the metropolitan region of Heidelberg, Germany. Patients suffering from COVID-19 were cared for on an outpatient basis and their health condition was monitored. Inclusion criteria of the study were: age ≥ 18 years, SARS-CoV-2 infection detected by RT- PCR, and informed consent for study participation. Exclusion criteria were age ≤ 18 years, inability to use the app, and absence of informed consent.

The usage of all samples and data was approved by the Ethics Commission of Heidelberg Medical University (S-324/2020) and all participants signed a written informed consent according to the declaration of Helsinki.

### Control Cohort

Total of 38 urine samples from the 26 in-house bank of healthy cohort participants were used as a part of "healthy" control. The usage of all samples and data was approved by the Ethics Commission of Murdoch University and all participants signed a written informed consent according to the declaration of Helsinki.

In addition, a second, control cohort from an in-house collected vaccination study was used (N=500+). Only the baseline points day zero and the 120 days after the first vaccination from the vaccination study totalling 39 samples from 24 individuals were used to create a "healthy" amalgamation of samples and exclude potential reactions of the participants to vaccine contraception. The usage of all samples and data was approved by the Ethics Commission of Murdoch University and all participants signed a written informed consent according to the declaration of Helsinki.

The combination of in-house healthy bank and vaccination cohort resulted in a total of 77 samples from 50 individuals for the urine analyses.

For the serum non-infected control cohort, 70 samples of 8 individuals were taken from the vaccination cohort at various time points before and after vaccination.

**Demographic Table A - Heidelberg cohort acute urine sample**

| | **All** | **Non-Hospitalised** | **Hospitalised** | **NA** |
|---|---|---|---|---|
| | N = 273 (100%)*1* | N = 186 (68%)*1* | N = 57 (21%)*1* | N = 30 (11%)*1* |
| Age (years) | 53.4 (14.6) | 52.7 (14.7) | 58.2 (14.1) | 48.8 (12.4) |
| NA | 1 | 0 | 0 | 1 |
| Gender | | | | |
| Female | 141 / 273 (52%) | 107 / 186 (58%) | 17 / 57 (30%) | 17 / 30 (57%) |
| Male | 131 / 273 (48%) | 79/186 (42%) | 40 / 57 (70%) | 12 / 30 (40%) |
| NA | 1 / 273 (0.4%) | 0/186 (0%) | 0 / 57 (0%) | 1 / 30 (3.3%) |
| BMI | 28.6 (6.2) | 28.2 (6.4) | 28.9 (5.7) | 30.5 (5.8) |
| NA | 29 | 17 | 8 | 4 |
| *1* Mean (SD); n / N (%) | | | | |

**Demographic Table B - Heidelberg cohort acute serum/plasma sample**

| | **ALL** | **Non-Hospitalised** | **Hospitalised** | **NA** |
|---|---|---|---|---|
| | N = 337 (100%)*1* | N = 231 (69%)*1* | N = 67 (20%)*1* | N = 39 (12%)*1* |
| Age (years) | 54.1 (15.0) | 52.7 (15.2) | 59.4 (13.6) | 53.4 (14.6) |
| NA | 2 | 0 | 0 | 2 |
| Gender | | | | |
| Female | 177 / 337 (53%) | 132/231 (57%) | 24 / 67 (36%) | 21 / 39 (54%) |
| Male | 158 / 337 (47%) | 99 / 231 (43%) | 43 / 67 (64%) | 16 / 39 (41%) |
| NA | 2/337 (0.6%) | 0/231 (0%) | 0 / 67 (0%) | 2 / 39 (5.1%) |
| BMI | 28.6 (6.0) | 28.4 (6.2) | 29.0 (5.5) | 29.4 (5.7) |
| NA | 30 | 17 | 8 | 5 |
| *1* Mean (SD); n / N (%) | | | | |
| Maximum collection time difference within the study participants was 371 days in serum and 85 days in urine. | | | | |

For NMR, urine healthy control with a total of 77 samples from 51 participants were collected. For LC-QQQ-MS, urine healthy control with a total of 39 samples were collected from 27 participants and 70 serum non-infected control samples from 6 participants.

**Demographic Table C - Healthy control cohort urine sample (NMR) and Non-infected cohort for urine and serum (LC-QQQ-MS)**

| | **Urine NMR** | **Urine LC-QQQ-MS** | **Serum LC-QQQ-MS** |
|---|---|---|---|
| | N = 51 (100%)*1* | N = 27 (100%)*1* | N = 6 (100%)*1* |
| Age (years) | 36.3 (11.2) | 34.2 (11.4) | 34.6 (8.1) |
| NA | 5 | 2 | 1 |
| Gender | | | |
| Female | 25/51 (49%) | 12/27 (44%) | 3/6 (50%) |
| Male | 24/51 (47%) | 13/27 (48%) | 3/6 (50%) |
| NA | 2/51 (3.9%) | 2/27 (7.4%) | |
| BMI | 24.0 (4.5) | - | 21.1 (2.6) |
| NA | 29 | 27 | 1 |

| | | | |
|---|---|---|---|
| *1* Mean (SD); n / N (%) | | | |

### Sample preparation, data acquisition and processing

### ¹H NMR sample preparation

Urine samples or urine after fractionation (see Preparative Liquid Chromatography) were thawed at 4 °C for 2 h then centrifuged for 15 minutes at 13000 g at 4 °C. Urine samples were prepared in 5 mm outer diameter SampleJet^{™} NMR tubes, following the recommended procedures for *in vitro* analytical and diagnostics procedures⁷ using 540 µL of Urine mixed with 60 µL phosphate buffer. Additionally, the samples were sonicated for 5 minutes at ambient temperature prior to analysis.

### ¹H NMR spectroscopy data acquisition for (IVDr) 600 MHz

NMR spectroscopic analyses were performed on a 600 MHz Bruker Avance III HD spectrometer, equipped with a 5 mm BBI probe and fitted with the Bruker SampleJet^{™} robot cooling system set to 5 °C. A full quantitative calibration was completed prior to the analysis using a protocol described elsewhere⁷. All IVDr methods were acquired at 300 K.

The standard one-dimensional (1D) experiments with solvent suppression (pp: noesygppr1d) were acquired with 32 scans (+4 dummy scans), 64k data points, relaxation delay of 4.0 s, and a spectral width of 20 ppm resulting in a total experiment time of 4 min 3s (according to the Bruker In Vitro Diagnostics research IVDR methods).

The spike-in experiments were performed with the same acquisition parameters as the standard 1D experiment at 300 and 320K. The experiments were performed on urine samples that showed "high" amounts of 1-4. The synthetic standards of 1-4 were prepared in 200 uL solutions (~1-5 mg/ml). After acquisition of the respective sample, increasing amounts (1-14 uL) of standard 1-4 were directly added to the sample. After thorough mixing the sample was measured again.

Time domain data were Fourier transformed and processed in automation using Bruker Topspin^{™} 3.6.2 and an exponential line broadening of 0.3 Hz was applied to the 1D water suppressed experiment,

### NMR spectroscopy data acquisition and processing for 800 MHz

The NMR spectra were acquired on a 800 MHz Bruker AvanceNeo spectrometer, equipped with a 5 mm TCI cryo-probe and fitted with a Bruker SampleJet^{™} robot cooling system set to 5 °C. The standard one-dimensional (1D) experiments with solvent suppression (pp: noesygppr1d) were acquired with 32-128 scans (+4 dummy scans), 128k data points, relaxation delay of 4.0 s, and a spectral width of 30 ppm resulting in a total experiment time of 4 min 3s (for 32 scans).

Selective 1D ¹H-TOCSY spectra were acquired with a standard sequence (pp: seldigpzs; with additional pre-saturation) using a z-filter adjusted according to the description made elsewhere⁸. Selective Gaussian pulses for refocusing were determined either using the Bruker ShapeTool or the Bruker interactive interface for selective experiments.. TD: 64k; SWₚₚₘ: 20; D1: 1.5-2.0 s; DS: 4; **NS:** 1k-16k; **Spinlock duration:** 120-180 ms.

Selective 1D ¹H NOESY spectra were acquired with a standard sequence (pp: selnogpzs.2; with additional pre-saturation) using a z-filter adjusted according to the description made elsewhere⁸. Selective Gaussian pulses for refocusing were determined either using the Bruker ShapeTool or the Bruker interactive interface for selective experiments. **TD:** 64k; SWₚₚₘ: 20; **D1:** 1.5-2.0 s; **DS:** 4; **NS:** 1k-16k; **Mixing time:** 800 ms.

¹H¹H COSY spectra spectra were acquired with a standard sequence (pp: cosygpprqf). TD F1: 4k-8k; **TD** F2: 1k-2k; **SWₚₚₘ** F1: 13; **SWₚₚₘ** F2: 13; **D1:** 2.0 s; **DS:** 16; **NS:** 2. Due to the long evolution times, some long range couplings are present in the COSY spectra.

¹H¹H _{longrange}COSY spectra spectra were acquired with a standard COSY sequence (pp: cosygpprqf) as a base, which was slightly modified to incorporate 2 delays (D6) before and after the second 90° hard pulse to allow for an additional coupling evolution of 400 ms . **TD** F1: 4k-8k; **TD** F2: 512-1k; **SWₚₚₘ** F1: 13; **SWₚₚₘ** F2: 13; **D1:** 2.0 s; **D6** = 200 ms **DS:** 16; **NS:** 4-20.

¹H¹H TOCSY spectra were acquired with a standard sequence (pp: dipsi2gpphzs; with additional pre-saturation or pp: dipsie2esgpphzs; usually pre-saturation is preferred due to the additional suppression of urea in urine samples, but sometimes the superior suppression of excitation sculpting was necessary for good spectral quality) using a z-filter adjusted according to the description made elsewhere⁸. Parameters: **TD** F1: 4k-16k; TD F2: 512-2k; **SWₚₚₘ** F1: 13; **SWₚₚₘ** F2: 13; **Spinlock duration:** 80-180 ms; **D1:** 2.0 s; **DS:** 32; **NS:** 2-16.

¹H¹H NOESY spectra were acquired with a standard sequence (pp: noesyesgpphzs) using excitation sculpting for water suppression and a *z*-filter adjusted according to the description made elsewhere⁸. Parameters: **TD** F1: 8k; **TD** F2: 512; **SW**ₚₚₘ F1: 12; **SW**ₚₚₘ F2: 12; **Mixing time:** 800 ms; **D1:** 2.0 s; **DS:** 16; **NS:** 32.

¹H¹³C HSQC spectra spectra were acquired with a standard sequence (pp: hsqcedetgpsisp2.3 or hsqcetgpisp2.3) including multiplicity editing and improved sensitivity scheme⁹. Parameters: **TD** F1: 4k; **TD** F2: 256-512; **SW**ₚₚₘ F1: 13; **SW**ₚₚₘ F2: 185; **¹J**_{(13C1H)}: 125 or 145 Hz; **D1:** 2.0 s; **DS:** 32; **NS:** 8-32.

¹H¹³C HMBC spectra were acquired with a standard three-fold low-pass J-filter sequence (pp: hmbcetgpl3nd; with additional pre-saturation). Parameters: TD F1: 4k; TD F2: 128-512; SWₚₚₘ F1: 13; SWₚₚₘ F2: 230; ¹J_{(13C1H)min}: 120 Hz; ¹J_{(13C1H)max}: 170 Hz; ⁿJ_{(13C1H)long range}: 5-8 Hz; D1: 2.0 s; DS: 16; NS: 72-256.

Time domain data were Fourier transformed and processed manually using Bruker Topspin^{™} 4.1.3, Bruker Topspin^{™} 4.1.4 and Bruker Topspin^{™} 4.2.0.

### LC-MS/MS Serum sample preparation

15 µL of sample was protein precipitated with 85 µL acetonitrile. Samples were vortex mixed for 10 minutes at 4°C and centrifuged for 30 minutes at 4°C. 80 µL supernatant was transferred to a 96-well plate and extracts were dried at room temperature before being reconstituted with 45 µL water. A pooled sample was from the total cohort for use as a pooled quality control (PQC) sample throughout analysis. Stock solutions of the standards were prepared at 1 mg/mL in water and diluted to solutions at 1 µg/mL for MS optimisation and retention time confirmation.

### LC-MS/MS experimental conditions

Reversed-phase chromatographic separation was performed using an Elute^{™} UHPLC system (Bruker Daltonics, Bremen, Germany) using a Waters Acquity BEH C18 column 1.7 µm, 50 x 2.1 mm. Mobile phase A comprised water with 0.5 % formic acid (v/v) and mobile phase B was acetonitrile with 0.5 % formic acid (v/v). A sample volume of 10 µL was injected. Gradient elution was performed at 0.4 mL/min with a starting composition of 5% B held for 0.5 min, increasing to 20 % B at 1.0 min, 95% B at 1.2 min and held for 0.5 min before returning to 5 % B at 1.8 min for equilibration until 2.3 min. The column oven was held at 60°C and autosampler at 6°C.

Mass spectrometric detection was performed on an EVOQ tandem quadrupole analyser (Bruker) operated in positive electrospray ionisation (ESI) mode. Source parameters were as follows: spray voltage 4000V; cone temperature 350°C; cone gas flow 20; heated probe temperature 350°C; probe gas flow 40; nebuliser gas flow 40. Multiple reaction monitoring was performed for the three analytes of interest with parameters detailed below. Data were acquired using HyStar v5.1 and processed using TASQ 2022 (Bruker Daltonics) resulting in peak area values that used for downstream analyses.

| Compound | RT (min) | Precursor ion (m/z) | Product ion 1 (m/z) | Collision energy (CE) | Product ion 2 (m/z) | Collision energy (CE) |
|---|---|---|---|---|---|---|
| ddhC (1) | 0.66 | 226.1 | 112.1 | 15 | 190.0 | 10 |
| ddhC-5'CA (2) | 0.60 | 240.1 | 112.0 | 14 | - | - |
| ddhU (3) | 0.70 | 227.1 | 148.0 | 12 | 115.1 | 9 |

### LC-MS/MS Urine sample preparation

10 µL of sample was diluted with 190 µL water (1:19 v:v) and vortex mixed. A pooled sample was pooled from the total cohort for use as a pooled quality control (PQC) sample throughout analysis.

### LC-MS/MS Urine experimental conditions

Reversed-phase chromatographic separation was performed using an Elute^{™} UHPLC system (Bruker) using a Waters Acquity HSS T3 column 1.7 µm , 100 ×2.1 mm. Mobile phase A comprised water with 0.5 % formic acid (v/v) and mobile phase B was acetonitrile with 0.5 % formic acid (v/v). A sample volume of 5 uL was injected. Gradient elution was performed at 0.3 mL/min with a starting composition of 0% B held for 0.2 min, increasing to 35 % B at 2.0 min, 95% B at 2.1 min and held for 0.4 min before returning to 0 % B at 2.6 min for equilibration until 3 min. The column oven was held at 40°C and autosampler at 6°C.

Mass spectrometric detection was performed on an EVOQ tandem quadrupole analyser (Bruker) operated in positive electrospray ionisation (ESI) mode. Source parameters were as follows: spray voltage 4000V; cone temperature 350°C; cone gas flow 20; heated probe temperature 350°C; probe gas flow 40; nebuliser gas flow 40. Multiple reaction monitoring was performed for the three analytes of interest with parameters detailed in table below. Data was acquired using HyStar v5.1 and processed using TASQ 2022 (Bruker Daltonics).

| Compound | RT (min) | Precursor ion (m/z) | Product ion 1 (m/z) | Collision energy (CE) | Product ion 2 (m/z) | Collision energy (CE) |
|---|---|---|---|---|---|---|
| ddhC (1) | 1.60 | 226.1 | 112.1 | 15 | 190.0 | 10 |
| ddhC-5'CA (2) | 0.98 | 240.1 | 112.0 | 14 | - | - |
| ddhC-5'Hcy (4) | 2.18 | 344.0 | 112.1 | 21 | - | - |
| ddhU (3) | 2.20 | 227.1 | 148.0 | 12 | 115.1 | 9 |

### Ultra High Resolution Mass Spectrometry

### Sample preparation

Samples (urine or plasma) were diluted 1:50 in Methanol/Water (9:1) 0.1% formic acid, cooled at -20°C for 10 minutes then centrifuged at 15,000 rpm for 10 minutes at 4°C. The supernatant was collected and transferred into a new vial prior to analysis.

### Fourier Transform Mass Spectrometry

A Bruker SolariX 7T Magnetic Resonance Mass Spectrometer was operated in quadripolar detection mode (2ω), magnitude and positive ionisation mode across the mass range *mlz* 107.5-2000 using Compass ftmsControl (ver. 2.3.0). A data size of 8M, 1M or 512k providing an estimated resolving power of 560k, 70k or 35k@400 *m*/*z,* 100-200 scans were averaged with an accumulation time of between 0.2-1s used. Atmospheric Pressure ionisation source settings were Capillary Voltage 4500V, Nebulize 1.0 bar, Dry Gas 4.0 l/min, Dry Temp. 200°C. Source optics, Capillary Exit 200V, Deflector Plate 220V, Funnel 1 150V, Skimmer 1 15V, Funnel RF Amplitude 120 Vpp; Octopole Frequency 5 MHz, RF Amplitude 350 Vpp; Collision Cell Voltage -2.0V, DC Extract Bias 0.2V, RF Freq. 2 MHz, Collision RF Amp. 1400.0 Vpp, Transfer Optics Time of Flight 0.4 ms, Frequency 6, RF Amplitude 350 Vpp were optimised for low molecular weight.

### Plasma Screening

For automated injection a Bruker PAL RSI liquid handling robot with 3 drawer Peltier Stack and, LCP 1 Robot Injection Arm and Cheminert Valve Drive, coupled to a Bruker Elute Pump and Column Oven was operated using Compass Hystar (ver. 5.1), a sample loop was loaded with 20 µl prior to injection using a direct infusion method over 3.5 minutes. Sample was delivered to the MRMS using 100% MeOH with the following solvent flow program 0-0.15 min 0.1 ml/min, 0.2-1.95 min 0.01 ml/min, 2.0-2.75 min 0.2 ml/min, 2.75-3.5 min 0.1 ml/min. The solvent was passed through a Waters 2.1mm, 0.2 µm filter frit and assembly prior to nebulisation. The instrument settings were modified, with the quadrupole set to *m*/*z* 238 using an isolation window of 30 Da, data size was set to 512k. Ions observed in plasma using high-throughput screening: ddhC (1) calc. C₉H₁₂N₃O₄ [M+H]⁺ *m*/*z* 226.082232, obs. *m*/*z* 226.08224 (ΔmDa 0.008, 0.04 ppm), ddhC-5'CA (2) calc. C₉H₁₀N₃O₅ [M+H]⁺ *m*/*z*240.061497, obs. *m*/*z*240.06172 (ΔmDa 0.22, 0.93 ppm).

### Direct infusion of Urine samples

For direct infusion of urine extracts samples were loaded into a Hamilton 250 µL syringe and infused at a rate of 10 µl/min using the same instrument settings. Instrument settings were modified with the quadrupole set to the expected ion mass (*m*/*z* 226,227,240, 250) with an isolation window of 5 Da and data size of 8M. Observed in urine using direct infusion: ddhC (1) (calc. C₉H₁₂N₃O₄ [M+H]⁺ *m*/*z226.082232,* obs. *m*/*z226.08229* (ΔmDa 0.058, 0.25 ppm), ddhC-5'CA (2) calc. C₉H₁₀N₃O₅ [M+H]⁺ *m*/*z*240.061497, obs. *m*/*z*240.06150 (ΔmDa 0.003, 0.01 ppm), ddhU (3) calc. C₉H₁₁N₂O₅ [M+H]⁺ *m*/*z*227.066248, obs. *m*/*z*227.06636 (ΔmDa 0.112, 0.49 ppm)

### Preparative Liquid Chromatography

Preparative and analytical optimisation scale HPLC were performed using a Waters Preparative HPLC system, using a Waters 2545 binary gradient module, equipped with a Waters 2998 diode array detector (DAD), a Waters AcquityQDa single quadrupole MS detector operating in ESI-(+) Mode, and a Waters 2767 sample manager unit as fraction collector. Analytical work was conducted using a Waters OBD C₁₈ reversed phase column (100 mm × 4.6 mm, 5 µm) utilising 10 µL injections, and with a flow rate of 1.46 mL/min, and preparative scale HPLC was undertaken with a Waters Sunfire OBD C₁₈ reversed-phase column (250 mm × 30 mm, 5 µm) with 600 µL injections at a flow rate of 14.0 mL/min. Solvents used were of LC-MS grade.

For preparative scale fractionation of urine from SARS-CoV-2 infected individuals, a sample of urine containing elevated levels of compounds of interest (30 mL) was concentrated *in vacuo,* reconstituted in deionised water (3.0 mL) and subsequently centrifuged (13,000 rpm, 10 min). The sample was decanted, and separated by preparative LC using an isocratic solvent system of 100% H₂O with 0.1% formic acid, held for 15 minutes, followed by a gradient of 0.0% MeCN: 100% H₂O to 5.0% MeCN: 95% H₂O with 0.1% formic acid over 15 minutes, followed by an increased gradient of 5.0% MeCN: 95% H₂O with 0.1% formic acid to 50% MeCN: 50% H₂O with 0.1% formic acid over 10 minutes. The 50% MeCN: 50% H₂O (with 0.1% formic acid) eluent was then held isocratically for 10 minutes and the column was then re-equilibrated to 100% H₂O with 0.1% formic acid over 15 minutes. Fractions were collected in 28.0 mL aliquots and concentrated to dryness *in vacuo.* Fractions were subsequently reconstituted in deionised water (1.0 mL) for NMR and MS analyses, affording semi-purified **8;** t_{R} = 0-4 minutes, **2;** t_{R} = 4-6 minutes, **1;** t_{R} = 6-8 minutes, **6;** t_{R} = 10-12 minutes, **4;** t_{R} = 18-20 minutes, **5;** t_{R} = 20-22 minutes, **3;** t_{R} = 22-24 minutes, and **7;** t_{R} = 26-28 minutes.

### Data preprocessing and bioinformatics

NMR 1D urine data was preprocessed in the statistical programming language R. ERETIC correction was applied to all spectra to ensure the observed intensities can be quantified. The residual water resonance peak (δ 4.6-6.0 ppm) was excised along with chemical shift regions where no signals were observed (δ < 0.4 ppm and δ > 9.5 ppm). Spectra were baseline-corrected using an asymmetric least-squares method and spectra were normalised using a probabilistic quotient method where the median spectrum is used as the reference.

Statistical evaluation was performed using principal component analysis (PCA) as an unsupervised method. Supervised multivariate analysis was performed using orthogonal projection to latent structures discriminant analysis (OPLS-DA) using the metabom8 package (version 0.2), obtainable at https://github.com/tkimhofer. Scores plots were constructed to show the differences between the controls and SARS-CoV-2 positive patients.

NMR lineshape fitting was completed in javaScript language using the packages brukerconverter (https://zenodo.org/record/7714532), nmr-processing (https://zenodo.org/record/7641749), github.com/cheminfo/filelist-utils and github.com/nmr-parser. The two overlapping doublets from ddhC and ddhU were modelled using four lines with estimated parameters of δ_{H} = 6.29 ppm, J = 1.9 Hz, and δ_{H} = 6.285 ppm, J = 1.9 Hz. ddhC-5'CA was modelled using two lines (doublet) with estimated parameters of δ_{H} = 6.3095 ppm, J = 2.0 Hz. Similarly, ERETIC and creatinine signal lineshape were modelled to a singlet (single line). Absolute concentrations were obtained by normalising the area of each signals to the area of the ERETIC peak. Concentrations of the ddhNs are then normalised to creatinine for reporting.

### Example 2: Acute SARS-CoV-2 infection is associated with a distinct urinary metabolic phenotype:

Paired urine and serum samples from a cohort of SARS-CoV-2 infected patients collected during the acute phase of the disease totalling 273 samples were profiled and qualitatively analyzed by ¹H NMR spectroscopy. The samples were collected between July 2022 and January 2023 in Heidelberg Hospital.The cohort was compared with urine samples from SARS-CoV-2 negative participants recruited from non-infected volunteers (n=50; with 77 samples in total due to multiple time points. All spectra were baseline corrected and normalized using probabilistic quotient normalization (PQN). Principal components analysis (PCA) indicated a variance largely unexplained by the first two principal components (PC1 & PC2), which led to compute up to 6 principal components. The key spectroscopic features driving the differentiation between the infected and non-infected models revealed: i) decreased excretion of hippuric acid in the infected samples; ii) presence of drugs such as acetaminophen and its downstream metabolites in the SARS-CoV-2 (+) spectra and iii) multiple unknown signals for PC4 in the chemical shift region ~δ_{H} 6.350-6.250 ppm that were present in the spectra of infected participants, but not in those in the control group (Fig. 1a-c). COVID-19 is already well described as a multisystem disease and liberal use of over the counter drugs is well known. Therefore, the presence of acetaminophen metabolites is not unexpected. While a complete modeling of these data is in progress, here we concentrate exclusively on the highly discriminating unknown signals. Indeed, an orthogonal projection to latent structure discriminant analysis (OPLS-DA) model focussing on the region of δ_{H} 6.360-6.220 ppm indicated clear differentiation of the control and infected sample groups with the OPLS-DA yielding a predictive model with AUROC of 0.96. Expansion of the region is given for healthy control samples and the SARS-CoV-2 (+) cohort in Fig. 1b and 1c respectively. Whereas the spectra of the control cohort are dominated by noise and no clear signals can be detected, two distinct pattern regions can be defined for the spectra of the infected participants at δ_{H} 6.320-6.310 ppm, δ_{H} 6.300-6.280 ppm and a third, minor pattern region can be detected at δ_{H} 6.270-6.250 ppm. From inspection of the median spectrum and statistical spectral distributions, the exact signal patterns of the metabolites of interest cannot be clearly discerned due to intersample peak shifts in urine. However, detailed evaluation of single spectrum revealed four key resonances: δ_{H} 6.29 ppm, J = 1.9 Hz; δ_{H} 6.31 ppm, J = 2.1 Hz; δ_{H} 6.29 ppm, J = 1.9 Hz; δ_{H} 6.26 ppm, J = 2.0 Hz stemming from four metabolites (Fig. 1d, 1-4). Comparison with in-house and public libraries yielded no match for the observed peaks, but their chemical shift in combination with their small coupling constants (~2 Hz) suggested an anomeric proton such as that commonly encountered at the CH-1' position of furanose moieties in nucleoside metabolites.

### Example 3: Structure elucidation of candidate biomarkers of SARS-CoV-2 infection:

Detailed ¹H NMR spectroscopic analysis of the samples yielding the highest intensity peaks from metabolite 1 (Fig. 1D), aided by combined homonuclear and heteronuclear2D NMR spectroscopy at 800 MHz, permitted assembly of the full spin system revealing a nucleoside-like structure with a cytosine moiety as nucleobase and a dehydrated furanose system. The NMR data were complemented by LC-ESI-MS analysis of the same urine sample used for NMR analysis monitoring for any precursor ions leading to the fragmentation of cytosine (*m*/*z* = 112.0505, calculated for [C₄H₆N₃O]⁺). Positive-mode HR-MS (QToF), and UHR-MS (FT-ICR) analysis of the most abundant metabolite compatible with the spectroscopic data presented above indicated a protonated molecular formula: [M+H]⁺ of C₉H₁₂N₃O₄: calculated *m*/*z* 226.082232, observed *m*/*z* 226.082240 (ΔmDa 0.008, 0.04 ppm) allowing for the putative molecular formula of metabolite **1** to be determined as C₉H₁₁N₃O₄. A rigorous database search of the ascertained molecular formula allowed for the assignment of **1** as the recently reported serum metabolite: 3'-deoxy-3',4'-didehydrocytidine (ddhC, 1), for which the reported ¹H and ¹³C NMR data were in good agreement with that obtained experimentally in urine. Using similar approaches, the structures of the remaining metabolites could be elucidated, identifying 3',5'-dideoxy-3',4'-didehydrocytidine-5'-carboxylic acid (ddhC-5'CA, **2),** 3'-deoxy-3',4'-didehydrouridine (ddhU, **3)** as previously unknown metabolites and 3',5'-dideoxy-3',4'-didehydrocytidine-5'-homocysteine (ddhC-5'Hcy, **4)** as a fourth metabolite, previously detected in a mouse urine model of severe *Plasmodium berghei* infection, that to the best of our knowledge has not been reported in humans (Fig. 1D). In addition, four additional metabolites, 3',5'-dideoxy-3',4'-didehydrouridine-5'-carboxylic acid (ddhU-5'CA, **5),** 3',5'-dideoxy-3',4'-didehydrocytidine-5'-thiomethylsulfoxide (ddhC-5'SO, **6),** 3',5'-dideoxy-3',4'-didehydrocytidine-5'-thiomethyl (ddhC-5'MeS, **7),** and 3',5'-dideoxy-3',4'- didehydrocytidine-5'-Methionine (ddhC-5'Met, **8)** could be elucidated from fractionated urine following preparative LC fractionation (Fig. 1d). In addition 2D ¹H¹H-TOCSY spectra indicated the presence of further unidentified ddhN compounds by following the ddhN inherent coupling pattern (ddhN spectroscopic signature) originating from the anomeric H-1' proton position (Fig. 1d and e). Finally, in order to unambigiously confirm the identity of metabolites **1-4,** synthetic samples of each were prepared and used in spiking experiments which confirmed the structure and presence of metabolites **1-4** in urine.

### Example 4: ddhNs 1-8 reveal an extended metabolic network actively expressed during viral infection:

Given the close structural homology between the elucidated biomarkers, a biosynthetic scheme could be deduced linking metabolites **1-8** to the viperin pathway (Fig. 2). As established in prior literature, CTP undergoes formal dehydration catalyzed by viperin with consumption of one unit of S-adenosylmethionine to afford ddhCTP. Subsequently, one could envision nucleotidase catalyzed hydrolysis to afford the elucidated biomarker ddhC **(1)** (detected at normalized urinary concentrations in excess of 20.0 µM/mM creatinine in acute phase patients). From here, oxidation of position C-5' of **1** (Fig. 2a) via a putative alcohol dehydrogenase and aldehyde dehydrogenase yielded the observed carboxylate moiety of ddhC-5'CA (2) (detected at normalized urinary concentrations on the order of 10.0 µM/mM creatinine), likely as a means of enzymatic clearance from the biological system. Hydrolysis of compound **1** via the action of a deaminase enzyme could also generate the observed metabolite ddhU **(3)** (detected at normalized urinary concentrations in excess of 10.0 µM/mM creatinine). Alternatively, it would be possible that UTP may be acting as a substrate for *Hs*-viperin *in vivo,* affording ddhUTP, with subsequent sequential hydrolysis of the triphosphate group producing compound **3.** The approximate 1000-fold substrate preference of CTP over UTP demonstrated by murine viperin *in vitro,* was noted at odds with the elevated concentrations of the downstream product **3** detected experimentally in urine (approx. 2:1, [1]:[3]). It would be possible that both proposed pathways towards metabolite **3** may be active under conditions of infection. In this regard the downstream substrate promiscuity of the viperin pathway *in vivo* suggests evolutionary adaptation to an expanded panel of viral pathogens, allowing for differential activity of the ddhN pharmacophore against an extended series of viral RNA polymerases. Subsequent sequential oxidation of ddhU **(3)** was likely to afford ddhU-5'CA (5), alternatively, the metabolite may also arise via hydrolysis of metabolite **2.**

In the case of the accumulated metabolite **4** (detected at concentrations up to 7.0 µM/mM creatinine), a ddhC **(1)** precursor with suitable leaving group, such as ddhCTP or ddhCDP, underwent a nucleophilic substitution reaction via an S_{N}2-type mechanism, with addition of methionine and concomitant loss of inorganic phosphate to produce the detected metabolite ddhC-5'Met **(8)** (Fig. 2d). From here, sulfonium dealkylation via the action of suitable biological nucleophiles afforded the observed major urinary metabolite **4.** The reaction sequence was presumably under enzymatic control, with parallel work in our laboratories establishing that S-adenosylmethionine Synthetase (MAT2A isoform) was capable of accomplishing this unfamiliar biochemistry. Dealkylation of **8** also yielded ddhC-5'MeS **(7),** with subsequent oxidation conducing to ddhC-5'SO **(6).** The detected relative concentration of **[4]:[7],** approx. 20:1, beared additional scrutiny, allowing to infer enzymatic, and not chemical, methyl group transfer by native biological methyltransferases. The exact biological functions and implications of this methyl group flux within the biological complex, in a pathway parallel yet distinct to SAM methyl group donation, are the subject of ongoing investigation.

### Example 5: Quantification of ddhNs derivatives in urine by NMR:

Motivated by the structural confirmation of compounds 1-3, the distribution of the compounds and their potential as markers for viral infections was investigated in urine by NMR. Therefore, the cohort of SARS-CoV-2 infected patient samples were quantitatively profiled during the acute phase of the disease totalling 273 samples and the control cohort with 77 samples. Metabolites **4-8** were not profiled because their urine concentration seemed generally to be too low for routine NMR analysis and were only detectable in a few concentrated samples.

Compounds **1-3** were directly quantified from the NMR spectra using a peak fitting approach of the anomeric CH-1' position that was adjusted according to the ERETIC signal and subsequently normalized to creatinine (Fig. 3a and 3b). The optimization yielded an approximate real limit of quantification of ~20 µM for all three compounds under the applied acquisition conditions (note that the concentrations in the text are normalised to creatinine which led to calculated values lower than 20 µM). The quantification and detection results are summarized in Table 1. By visual inspection of the spectral data, a total of 239 (88%) of SARS-CoV-2 (+) samples were observed to contain ddhC **(1),** of which 151/273 (55%) samples could be reliably quantified. Similarly, 199 (73%) of ddhU **(3)** could be detected, of which 151/273 (55%) were quantified (Fig. 3a). ddhC-5'CA **(2)** was found in fewer samples, being present in 84/273 (31%) samples with 101 (37%) more samples containing compound **2** at levels below the limit of quantification bringing the total of samples to 68%. Further sub-dividing the SARS-CoV-2 (+) cohort into hospitalised, non-hospitalised and not assigned (NA) patients revealed that 1 and 3 were successfully quantified in 40/57 (70%) of the hospitalised and **1** could be found in 16 more samples above the limit of detection totaling the presence of **1** to 56/57 (98%) of the hospitalised samples. In addition, **1-3** were quantified in 92/186 (49%) of the non-hospitalised and in 19/30 (63%) of the NA patients. Distinction by hospitalised, non-hospitalised and NA patients identified compound 3 to be present in 36/57 (63%) of the hospitalised, 44/186 (24%) for the non-hospitalised and 6/30 (20%) of the NA patients. Compounds **1-3** could not be detected in any of the 77 control samples; even after manual inspection of every spectrum (Fig. 3a-c). These results suggested that nucleosides **1-3** were only expressed at NMR detectable concentrations under disease conditions and that there was a clear prevalence for detection in hospitalized patients, i.e. more severe/sick study participants.

**Table 1: Number and percentage of samples in which the new markers were detected and quantified**

| | All acute patients | | | Only hospitalized patients | | | Controls | | Method/ Matrix |
|---|---|---|---|---|---|---|---|---|---|
| | N | Detected | Quantified | N | Detected | Quantified | N | Detected | |
| ddhC (1) | 273 (100%) | 239 (88%) | 151 (55%) | 57 (100%) | 56 (98%) | 40 (70%) | 77 (100%) | 0 (0%) | NMR/ urine |
| ddhC-5'CA (2) | | 185 (68%) | 84 (31%) | | 46 (81%) | 36 (63%) | | 0 (0%) | |
| ddhU (3) | | 199 (73%) | 151 (55%) | | 51 (89%) | 40 (79%) | | 0 (0%) | |
| ddhC (1) | 273 (100%) | 269 (99%) | 269 (99%) | 57 (100%) | 56 (98%) | 56 (98%) | 39 (100%) | 38 (97%) | MS/ (QQQ) urine |
| ddhC-5'CA (2) | | 264 (97%) | 264 (97%) | | 56 (98%) | 56 (98%) | | 38 (97%) | |
| ddhU (3) | | 262 (96%) | 262 (96%) | | 56 (98%) | 56 (98%) | | 38 (97%) | |
| ddhC (1) | 524 (100%) | 515 (98%) | 515 (98%) | 117 (100%) | 115 (98%) | 115 (98%) | 70 (100%) | 20 (29%) | MS/ (QQQ) serum |
| ddhC-5'CA (2) | | 286 (55%) | 286 (55%) | | 77 (66%) | 77 (66%) | | 3 (4%) | |
| ddhU (3) | | 405 (77%) | 405 (77%) | | 102 (87%) | 102 (87%) | | 2 (3%) | |

To further understand the association of compounds **1-3** to each other, linear regressions comparing all three were performed (Fig. 3c) demonstrating a prominent correlation (R² = 0.66) between ddhC **(1)** and ddhU **(3),** but only modest correlations between dhhC (1) or ddhU **(3)** against the carboxylic acid derivative ddhC-5'CA (2) (R² = 0.28 and 0.37 respectively), potentially indicating that **1** and **3** likely followed the same metabolic step i.e. conversion of CTP and UTP to ddhCTP and ddhUTP respectively by the viperin enzyme, whereas ddhC-5'CA **(2)** was most likely metabolized downstream in the pathway and that the conversion rate via oxidative metabolism may differ amongst individual study participants.

### Time trajectories indicate rapid urinary excretion of ddhNs from the system:

For 12% of the participants in the original cohort, none of the nucleoside compounds **1-3** could be detected in the urine samples . This could be due to short half life of the compounds in the system, as most samples were taken several days into the infection period, and in some instances more than a week after infection. In order to further characterize the time profile of production and excretion of these candidate nucleoside biomarkers, the urine time course was tracked over the first seven days post-infection, for six individuals newly infected with SARS-CoV-2 as determined by a positive PCR/RAT test. For all six participants, nucleosides **1-3** could be detected from the first available time point, indicating a very rapid response. Subsequent timed collections exhibited a rapid decrease in concentration of all metabolites. After 4-5 days of infection, the urinary concentrations of compounds **1-3** were close to or below the NMR limit of detection.

### Quantification of ddhNs derivatives in urine by LC-QQQ-MS establishes basal viperin expression in healthy controls:

To put this hypothesis to test, the ddhNs **1-3** were also profiled by targeted LC-QQQ-MS analysis in urine, in a process parallel to that performed via NMR analysis for the same samples. For the LC-QQQ-MS assay, **1-3** could be detected in 99% of all SARS-CoV-2 infected subjects and 98% of the control cohort (Table 1), whereas the average concentration of all three compounds was at least one order of magnitude higher (13-78 times) in diseased subjects compared to controls.

Importantly, the results still displayed a clear distinction between diseased and non-diseased subjects. Additionally, this indicated that the LC-QQQ-MS assay sensitivity was high enough to detect and confirm basal expression of the viperin enzyme within the system via accumulation of **1-3** in the urine of healthy subjects.

Linear regression of compounds **1-3** in similar fashion to the NMR analysis was carried out on the LC-QQQ-MS data. The relation of ddhC **(1)** with ddhU **(3)** showed a higher correlation than in urine (R² = 0.80 v.s. 0.66 in urine) and also the correlation ddhC **(1)** and ddhU **(3)** against the carboxylic acid derivative ddhC-5'CA **(2)** were increased significantly (ddhC/ddhC-5'CA R² = 0.68; 0.28 in urine; ddhu/ddhC-5'CA R²=0.77; urine 0.37) compared to the NMR data. This was probably due to the additional data and inclusion of low ddhN concentrations at the basal expression level, which might be regulated to a higher degree than under disease conditions.

### Example 6: Detection of ddhNs in serum by targeted LC-QQQ-MS:

To ascertain if ddhC-5'CA **(2)** and ddhU **(3)** are also present in blood; in addition to ddhC **(1),** which has been reported in serum previously, the corresponding serum samples were analyzed. NMR spectroscopy could not detect any of the three compounds in serum due to its inherent insensitivity, but all three metabolites could be successfully detected in serum with the targeted LC-QQQ-MS assay. ddhC **(1)** was present in 98% of all SARS-CoV-2 (+) samples, whereas 2 and **3** were successfully detected in most samples at 55% and 77% respectively (Table 1). Compounds **1-3** could also be detected in some non-infected control samples (Table 1), but the average peak area of **1-3** in controls was determined to be at least one order magnitude lower compared to infected subjects and is in line with the results in urine by NMR and LC-QQQ-MS.

The detected amount of **1-3** in healthy controls was possibly due to the basal expression of interferons and downstream viperin expression as described above.

Linear regression of compounds **1-3** was carried out in addition in order to investigate differences between urine and serum dataFig.. The relation of ddhC **(1)** with ddhU **(3)** showed a correlation of R² = 0.74 and correlations of ddhC **(1)** and ddhU **(3)** against the carboxylic acid derivative ddhC-5'CA (2) were dhC/ddhC-5'CA R² = 0.45 and ddhu/ddhC-5'CA R²=0.39 respectively. Similar to urine by NMR and LC-QQQ-MS, the relation of ddhC **(1)** to ddhU **(3)** showed the highest correlation of the set. The lower correlations of dhC/ddhC-5'CA (R² = 0.45) and ddhu/ddhC-5'CA (R²=0.39) were in alignment with the NMR urine data. Just like the urine NMR, the LC-QQQ-MS serum assay mostly picked out sample concentrations above the basal level explaining the deviation to the LC-QQQ-MS urine data.

Infection with SARS-CoV-2 provokes a complex range of immunological and metabolic perturbations across multiple organ systems, which manifest as observable abnormalities across multiple biochemical pathways. Some of these disruptions result from activation of innate and adaptive immune response and offer the opportunity for rapid detection, severity prognosis prediction and patient stratification in a clinical setting, whilst other disruptions are related to longer term impacts on metabolism. In this study, extensive, cohort wide metabolic profiling analyses of SARS-CoV-2 infected individuals allowed for the detection of a key spectroscopic signature (Fig. 1) attributed to the anomeric methine CH-1' of a class of compounds that were refer to as deoxydidehydronucleosides (ddhNs) or 3'(,5'-di)deoxy-3',4'-didehydro furanose derivatives respectively. Interestingly, this key signature appeared in a region of the ¹H NMR urine spectrum typically devoid of other resonances, highlighting its potential value as a diagnostic biomarker, in particular using low field cost-effective instruments. The structures of metabolites 1-4 (Fig. 2) were elucidated directly from unfractionated urine from SARS-CoV-2 infected participants through a combination of NMR spectroscopic and mass spectrometric analysis, and further validated via total synthesis of the proposed structures with subsequent spiking of the chemical standards into the relevant biological fluids, allowing for a rigorous MSI level-one annotation of all four metabolites. In addition, four further metabolites **(5-8,** Fig. 2) could be elucidated from fractionated urine. At least four additional minor metabolites with structural features seemingly in common with the reported compounds **1-8** were also detected by NMR and MS during the course of the investigations into the urine of SARS-CoV-2 infected patients, the full structural elucidation of which remains ongoing (Fig. 1e). The most abundant compounds **1-3** could also be successfully quantified in serum samples of the same SARS-CoV-2 infected cohort by means of LC-MS profiling.

A biosynthetic schema linking compounds **1-8** to the extended viperin pathway and the known metabolites cytidine triphosphate (CTP) and uridine triphosphate (UTP) is proposed (Fig. 2). Nucleotidase hydrolysis of ddhCTP is suggested to afford ddhC **(1),** with subsequent hydrolysis affording ddhU **(3),** alternatively UTP may be acting as an *in vivo* substrate for viperin, to afford ddhUTP, with hydrolysis affording **3.** Oxidation at position C-5' of the relevant scaffolds is proposed to afford the observed metabolites **2** and **5,** likely as a means of clearance from the biological system. In addition, the presence of ddhU-5'CA **(5)** suggests pyrimidine "pairing" across the metabolite series, offering insight into the possible identity of additional metabolites within the ddhN pathway that currently remained below the conventional limit of detection. Of note, under no conditions were the known immediate products of the viperin pathway: ddhCTP, ddhCMP and ddhUTP⁵, observed in any of the clinical samples (urine and serum) under investigation, likely due to the decreased plasma-membrane permeability of the triphosphorylated metabolites leading to limited circulation through the measured biological fluids, as well as the action of promiscuous nucleotidases present in the cytosol and in plasma leading to a short biological halflife of the phosphorylated species. In this respect metabolites **1** and **3** represent potential extracellular reservoirs of the biologically active ddhCTP and ddhUTP pharmacophores, as well as a means of transport via passive diffusion from the site of infection to the remainder of the biological system.

The presence of the homocysteinylated adduct: ddhC-5'Hcy **(4),** accumulated at considerable concentrations in urine is of significant interest, seemingly standing outside of the scope of traditional excretory xeno-metabolism, via conjugation to cysteine/glutathione and the mercapturate pathway. Methionine conjugation to ddhCTP has been observed, yielding the detected intermediate metabolite ddhC-5'Met **(8),** with subsequent dealkylation yielding the major product **4,** as well as the minor metabolite ddhC-5'MeS **(7),** and oxidation of **7** producing ddhC-5'SO **(6).** Conjugation of methionine to the ddhN scaffold and subsequent enzymatic methyl group donation, as observed via elevated accumulation of the downstream product **4** versus the presumed chemical dealkylation products **6** and **7** may have profound implications for system-wide response to viral infection catalyzed via global perturbations to multiple biochemical pathways.

The major ddhN metabolites **1-3** were found to accurately distinguish infection with SARS-CoV-2 from healthy controls in urine (Table 1 and Fig. 3a). Quantification was performed using a peak fitting approach only when overlap prevented the use of simpler integration, or a targeted LC-QQQ-MS assay using the available standard **1-3.** The anomeric protons at position H-1' of all three compounds resonate in a frequency range that is typically void of other peaks (Fig. 1b and c). Hence, quantification of the anomeric proton peak from compounds **1-3** circumvents the inherent spectral convolution problem of urine NMR spectra. NMR spectroscopy presents several advantages as a metabolic profiling platform. These include a simple and fast sample preparation process, an extended linear dynamic range, and robust instrumentation. The metabolic analysis of urine over alternative bio-fluids, e.g. serum, plasma, bronchoalveolar lavage fluid or seminal fluid, represents additional advantages, namely patient compliance, and the ease of sample collection obviating the need for collection by phlebotomists or other trained professionals. A recent report by Sriskandan and co-workers reported the detection of ddhC (1) in the serum and plasma of SARS-CoV-2 infected individuals experiencing acute viral infection following untargeted HILIC-QToF-MS analysis, with the metabolite shown to outperform white cell counts, lymphocyte counts, and CRP as a viral biomarker.⁶ The authors additionally showed that levels of 1 were 36-fold higher in patients with viral infections, including SARS-CoV-2, in comparison to bacterial infections⁶. The observation by NMR spectroscopy of metabolites **1-8** in the urine of SARS-CoV-2 infected patients represents a number of advantages over the former mass spectrometry based assay, as detailed.

The metabolites disclosed herein represent an auspicious development in the search for a general diagnostic assay for acute viral infection with real-world clinical utility. Although the cohorts analyzed in this study were restricted to patients infected with SARS-CoV-2, it is expected that the newly observed biomarkers may prove useful in diagnosing alternative acute viral infections, and additional work to this end is being conducted in our laboratories and will be reported in due course. A rapid diagnostic assay built around the disclosed metabolites, facilitated by the ease of urinary bio-fluid collection, would find ready application in intensive care units, allowing for the expeditious stratification of patients experiencing acute inflammatory distress. In this regard one can draw analogy to the currently employed procalcitonin assay, used for the diagnosis of bacterial bronchitis, pneumonia, COPD exacerbation and severe bacterial sepsis. It is envisioned that an expedient assay allowing for the robust differentiation of viral infection from other sources of acute inflammation could provide tangible benefits in a clinical setting where resource and time allocation are essential to patient prognosis.

### Cited Literature

1. Nicholson, J. K. & Wilson, I. D. High resolution proton magnetic resonance spectroscopy of biological fluids. Prog. Nucl. Magn. Reson. Spectrosc. 21, 449-501 (1989).
2. De Clercq, E. A 40-year journey in search of selective antiviral chemotherapy. Annu. Rev. Pharmacol. Toxicol. 51, 1-24 (2011).
3. Vangeel, L. et al. Remdesivir, Molnupiravir and Nirmatrelvir remain active against SARS-CoV-2 Omicron and other variants of concern. Antiviral Res. 198, 105252 (2022).
4. Gizzi, A. S. et al. A naturally occurring antiviral ribonucleotide encoded by the human genome. Nature 558, 610-614 (2018).
5. Soumi Ghosh and E. Neil. G. Marsh; "Viperin: An ancient radical-SAM enzyme finds its place in modern cellular metabolism and innate immunity" J. Biol. Chem. Vol. (2020) 295, Issue 33, p.11513-11528;
6. Mehta, R. et al. Antiviral metabolite 3'-deoxy-3',4'-didehydro-cytidine is detectable in serum and identifies acute viral infections including COVID-19. Med3, 204-215.e6 (2022).
7. Andersen, K. G., Rambaut, A., Lipkin, W. I., Holmes, E. C. & Garry, R. F. The proximal origin of SARS-CoV-2. Nat. Med. 26, 450-452 (2020).
8. Kimhofer, T. et al. Integrative Modeling of Quantitative Plasma Lipoprotein, Metabolic, and Amino Acid Data Reveals a Multiorgan Pathological Signature of SARS-CoV-2 Infection. J. Proteome Res. 19, 4442-4454 (2020).
9. Al-Aly, Z., Bowe, B. & Xie, Y. Long COVID after breakthrough SARS-CoV-2 infection. Nat. Med. 28, 1461-1467 (2022).

## Claims

1. A method for diagnosing a viral infection or a disease associated therewith in a subject comprising the steps of:
(a) determining in a sample of said subject the amount of at least one biomarker selected from the group of 3'(,5'-di)deoxy-3',4'-didehydro furanose derivatives of formula (I) 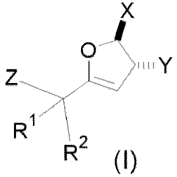 , wherein
R¹, R² are each a hydrogen atom or together form a (double bonded) oxygen atom;
X is selected from the group consisting of cytosine, uracil and thymine;
Y is selected from the group consisting of hydrogen atom, hydroxyl group, -O-SO₃H group, and -O-PO₃H₂ group; and
Z is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃ group, -SOCH₃ group, -S-(CH₂)₂-CH(NH₂)-CO₂H group and -S⁺(CH₃)-(CH₂)₂- CH(NH₂)-CO₂H group;
with the exception of 3'-deoxy-3',4'-didehydro-cytidine (ddhC); and
(b) comparing the amount(s) of the at least one biomarkers determined in (a) with a reference, thereby diagnosing a viral infection.

2. The method of claim 1, wherein the at least one biomarker is selected from the group consisting of 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'-homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), 3',5'-dideoxy-3',4'-didehydro-cytidine-5'-thiomethyl (ddhC-5'MeS) and 3',5'-dideoxy-3',4'- didehydrocytidine-5'-methionine (ddhC-5'Met), preferably selected from the group consisting of ddhC-5'CA, ddhU and ddhC-5'Hcy, more preferably the at least one biomarker is ddhC-5'CA and/or ddhU.

3. The method of claim 2, wherein the method further comprises determining the amount of 3'-deoxy-3',4'-didehydro-cytidine (ddhC) in said sample of the subject in step (a) and comparing the amount of ddhC to a reference in step (b).

4. The method of claims 1 to 3, wherein determining the amount of the at least one biomarker in (a) is done by a method selected from the group consisting of mass spectrometry (MS), nuclear magnetic resonance (NMR) spectroscopy, liquid chromatography (LC), gas chromatography (GC) and a combination of two or more of these methods, wherein preferably determining the amount of the at least one biomarker in (a) is done by NMR spectroscopy, more preferably by ¹H-NMR spectroscopy, more preferably ¹H-NMR spectroscopy focused on at last one of the chemical shift regions δH in the range of from 7.50 to 7.35 ppm, in the range of from 6.35 to 6.23 ppm, in the range of from 6.05 to 5.83 ppm, in the range of from 5.60 to 5.35 ppm, in the range of from 4.90 to 5.15 ppm, and in the range of from 4.35 to 3.26 ppm.

5. The method of any one of claims 1 to 4, wherein the sample of the subject is a tissue sample or a body fluid sample, wherein the body fluid sample is preferably selected from the group consisting of: spinal fluid, lymph, blood, preferably whole blood, plasma or serum, gastrointestinal fluids, preferably saliva, gastric acid, pancreas fluid or bile, urine, and sweat, wherein the body fluid sample is more preferably a urine sample.

6. A method for diagnosing a viral infection or a disease associated therewith in a subject comprising the steps of:
(A) determining in a sample of said subject the amount of 3'-deoxy-3',4'-didehydro-cytidine (ddhC); and
(B) comparing the amount of the ddhC determined in (A) with a reference, thereby diagnosing a viral infection;
wherein the determination in (a) is done by NMR spectroscopy and the sample of the subject is a urine sample of said subject.

7. The method of any one of claims 1 to 6, wherein the subject is a mammal, preferably a human.

8. The method of any one of claims 1 to 7, wherein the method is an *ex vivo* method.

9. The method of any one of claims 1 to 8, wherein the viral infection is a viral infection associated with a RNA-dependent RNA polymerase, preferably a viral infection associated with a RNA-dependent RNA polymerase, which is inhibitable by viperin, more preferably a viral infection selected from the group consisting of Zika virus infection, West Nile virus infection, Dengue virus infection, Hepatitis-C-Virus infection, and SARS-CoV-2 infection, more preferably the viral infection is a SARS-CoV-2 infection;
and/or
wherein the associated disease is Corona Virus Disease 2019 (COVID-19).

10. The method of any one of claims 1 to 9, wherein said reference is derived from the amount of the at least one biomarker from a subject or group of subjects known to suffer from the viral infection and/or the associated disease to be diagnosed.

11. A method for identifying whether a subject is in need of a viral infection therapy comprising the steps of the method of any one of claims 1 to 10 and the further step of identifying a subject in need of a viral infection therapy if said subject is diagnosed to suffer from viral infection or a disease associated therewith.

12. A diagnostic means for use in diagnosing a viral infection or a disease associated therewith, said diagnostic means comprising at least one biomarker selected from the group of 3'(,5'-di)deoxy-3',4'-didehydro furanose derivatives of formula (I) 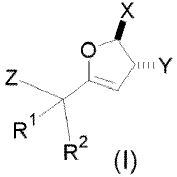 , wherein
R¹, R² are each a hydrogen atom or together form a (double bonded) oxygen atom;
X is selected from the group consisting of cytosine, uracil and thymine;
Y is selected from the group consisting of hydrogen atom, hydroxyl group, -O-SO₃H group, and -O-PO₃H₂ group; and
Z is selected from the group consisting of hydrogen atom, hydroxyl group, -O-SO₃H group, -O-PO₃H₂ group, -O-PO₃PO₃H₃ group, -O-PO₃PO₃PO₃H₄- group, -SCH₃ group, -SOCH₃ group, -S-(CH₂)₂-CH(NH₂)-CO₂H group and -S⁺(CH₃)-(CH₂)₂- CH(NH₂)-CO₂H group;
with the exception of 3'-deoxy-3',4'-didehydro-cytidine (ddhC).

13. A device for diagnosing a viral infection or a disease associated therewith in a sample of a subject comprising:
(i) an analyzing unit for the sample of the subject comprising a detector for the amounts of at least one biomarker selected from the group of 3'(,5'-di)deoxy-3',4'-didehydro furanose derivatives of formula (I) 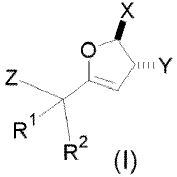 , wherein
R¹, R² are each a hydrogen atom or together form a (double bonded) oxygen atom;
X is selected from the group consisting of cytosine, uracil and thymine;
Y is selected from the group consisting of hydrogen atom, hydroxyl group, -O-SO₃H group, and -O-PO₃H₂ group; and
Z is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃group, -SOCH₃group, -S-(CH₂)₂-CH(NH₂)-CO₂H group and -S⁺(CH₃)-(CH₂)₂- CH(NH₂)-CO₂H group;
with the exception of 3'-deoxy-3',4'-didehydro-cytidine (ddhC); said detector allowing for the determination of the amounts of the biomarkers of the said group of biomarkers in the sample; and operatively linked thereto,
(ii) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established.

14. A kit for identifying a subject suffering from a viral infection or a disease associated therewith, the kit comprising
(i) a reference sample of at least one biomarker selected from the group of 3'(,5'-di)deoxy-3',4'-didehydro furanose derivatives of formula (I) 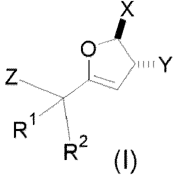 , wherein
R¹, R² are each a hydrogen atom or together form a (double bonded) oxygen atom;
X is selected from the group consisting of cytosine, uracil and thymine;
Y is selected from the group consisting of hydrogen atom, hydroxyl group, -O-SO₃H group, and -O-PO₃H₂ group; and
Z is selected from the group consisting of hydrogen atom, hydroxyl group, -O-SO₃H group, -O-PO₃H₂ group, -O-PO₃PO₃H₃ group, -O-PO₃PO₃PO₃H₄ group, -SCH₃group, -SOCH₃group, -S-(CH₂)₂-CH(NH₂)-CO₂H group and -S⁺(CH₃)-(CH₂)₂- CH(NH₂)-CO₂H group;
and/or
(ii) at least one binding agent capable of binding at least one biomarker selected from the group of 3'(,5'-di)deoxy-3',4'-didehydro furanose derivatives of formula (I).

15. Use of at least one biomarker selected from the group comprising 3'-deoxy-3',4'-didehydro-cytidine (ddhC), 3',5'-dideoxy-3',4'-didehydrocytidine-5'-carboxylic (ddhC-5'CA), 3'-deoxy-3',4'-didehydro-uridine (ddhU), 3',5'-dideoxy-3',4'- didehydrocytidine-5'homocysteine (ddhC-5'Hcy), 3',5'-dideoxy-3',4'-didehydrouridine -5'-carboxylic acid (ddhU-5'CA), 3',5'-dideoxy-3',4'-didehydrocytidine-5'-thiomethylsulfoxide (ddhC-5'SO), and 3',5'-dideoxy-3',4'-didehydrocytidine-5'-thiomethyl (ddhC-5'MeS) for diagnosing a viral infection, preferably a SARS-CoV-2 infection, or a disease associated therewith, preferably Corona Virus Disease 2019 (COVID-19), by NMR spectroscopy in a urine sample of a subject.
